# EUROPÄISCHE PATENTSCHRIFT

(11) **EP 2 316 965 B1**
(45) Veröffentlichungstag und Bekanntmachung des Hinweises auf die Patenterteilung: **30.04.2014**
(21) Anmeldenummer: 10009216.2
(22) Anmeldetag: 04.05.2007
(51) Int. Cl.: C12Q 1/68

(54) **Einführung von Sequenzelementen in Nukleinsäuren**
Introduction of sequence elements in nucleic acids
Introduction d'éléments de séquence dans des acides nucléiques

(30) Priorität: 05.05.2006 DE 102006020885
(43) Veröffentlichungstag der Anmeldung: 04.05.2011
(62) Teilanmeldung aus: 07107494.2
(73) Patentinhaber: Qiagen GmbH, 40724 Hilden (DE)
(72) Erfinder: Korfhage, Christian, 40764 Langenfeld (DE); Engel, Holger, 40724 Hilden (DE); Löffert, Dirk, 40589 Düsseldorf (DE)

(56) Entgegenhaltungen:
- WO-A-00/70095
- WO-A-2004/009814
- US-A1- 2004 023 271

## Beschreibung

Die vorliegende Erfindung betrifft Verfahren zur Einführung von Sequenzelementen (auch Tag-Sequenzen genannt) in Nukleinsäuren.

Um in eine entstehende Nukleinsäure, insbesondere am 3'-Ende der entstehenden Nukleinsäure, ein Sequenzelement (eine Tag-Sequenz) einzuführen, ist es möglich eine Nukleinsäure-Synthese des Gegenstranges der entstehenden Nukleinsäure oder eine Ligation eines sog. Adaptors/Linkers durchzuführen. Diese bekannten Verfahren sind zeitaufwendig und umständlich. Derzeit sind drei verschiedene Methoden zur Einführung spezifischer Tag-Sequenzen an 3'-Enden von Nukleinsäuren bekannt, die als gemeinsame Schritte enthalten, dass (a) ein Gegenstrang zur bereits in einer Polymerase-Reaktion synthetisierten Nukleinsäure hergestellt wird oder (b) eine zweite enzymatische Reaktion benötigt wird, um eine Tag-Sequenz an eine Nukleinsäure anzuhängen. Dabei handelt es sich um folgende drei Verfahren:
1) Schon früh wurde eine Template-unabhängige DNA-Polymerase genutzt, um am 3'-Ende einer DNA ein Homopolymer anzuhängen. Für diese Methode ist eine Template-unabhängige Polymerase notwendig, die das Homopolymer in einer unabhängigen Reaktion polymerisiert. Diese Methode kann dadurch erweitert werden, dass ein Oligonukleotid, das eine Sequenz enthält, die komplementär zum angehängten Homopolymer ist, die Synthese des Gegenstranges spezifisch starten kann. Ursprünglich wurde diese Methode bei cDNA-Klonierungen benutzt (Okayama H. & Berg P., High-Efficiency Cloning of Full-Length cDNA, Mol. Cell. Biol. 1982, 161 - 170). Heutzutage wird diese Methode auch für die lineare RNA-Amplifikation eingesetzt.
2) Des weiteren ist es bekannt, so genannte "template-swichting primer" einzusetzen, die eine spezifische Tag-Sequenz am CAP-Ende einer mRNA einfügen (Matz M., Shagin D., Bogdanova E., Britanova O., Lukyanov S., Diatchenko L. & Chenchik A., Amplification of cDNA ends based on template-switching effect and step-out PCR, Nucl. Acid Res., 1999, Vol. 27, No. 6, 1558 - 1560; US-Patent Nr. 5,962,272). Diese Methode ist unmittelbar verknüpft mit der Eigenschaft einer bestimmten Reversen Transkriptase (MMLV-RNase H minus), am CAP-Ende einer RNA einige Cytosin-Nukleotide Template-unabhängig anzuhängen. Dieses Anhängen von Nukleotiden entspricht einem Polymer-Tailing wie bereits unter 1) beschrieben, das jedoch hier durch die Reverse Transkriptase durchgeführt wird. Die Cytosin-Nukleotide können für die Hybridisierung eines Oligonukleotids, das am 3'-Ende G-Basen aufweist, genutzt werden. Diese Methode wird z.B. bei cDNA-Klonierungen verwendet.
3) Schließlich kann der Gegenstrang auch durch Primer gestartet werden, die von außen zugefügt werden. Diese Primer-Oligonukleotide können z.B. neben einer Zufallssequenz an ihrem 5'-Ende eine spezifische Sequenz (Tag-Sequenz) enthalten, wodurch am 5'-Ende in den neu synthetisierten Strang eine Tag-Sequenz eingeführt wird. Diese Form der Synthese nutzt zwar einen Primer mit Tag-Sequenz, aber die Gegenstrang-Synthese ist in jedem Fall notwendig, um eine zur Tag-Sequenz komplementäre Sequenz am 3'-Ende einzuführen. Diese Methode wird z.B. bei der cDNA-Klonierung oder bei Amplifikationsreaktionen verwendet.

So beschreibt beispielsweise die US-Patentanmeldung Nr. 2003/0143599 von Makarov et al. ein Verfahren zur Herstellung eines DNA-Moleküls, bei welchem ein DNA-Molekül gewonnen wird, das anschließend in Zufallsabschnitte zerstückelt wird. Dann wird ein Primer mit einer im wesentlichen bekannten Sequenz an mindestens eines der gewonnenen Fragmente angehängt, um mit Primem verbundene Fragmente ("primer-linked fragments") herzustellen, die anschließend amplifiziert werden. Allerdings wird bei dem Verfahren nach der vorgenannten US-Patentanmeldung der Primer am 3'-Ende des entstehenden DNA-Fragments über so genanntes "Tailing" oder eine Adaptor-Ligation eingeführt.

Schließlich beschreibt ein Artikel (Wharam S.D., Marsh P., Lloyd J.S., Ray T.D., Mock G.A., Assenberg R., McPhee J.E., Brown P., Weston A. and Cardy D.L.N., Specific detection of DNA and RNA targets using a novel isothermal nucleic acid amplification assay based on the formation of a three-way junction structure, Nucleic Acids Research, 2001, Vol. 29, No. 11 e54, 1 - 8) den Nachweis bestimmter Sequenzen in DNA oder RNA unter Verwendung von Oligonukleotiden, bei denen jedoch kein Matrizenwechsel ("Template-Switch") stattfindet. Stattdessen wird hier ein Oligonukleotid ("Extension Probe" genannt) verlängert, das sowohl an der Zielsequenz als auch an der "Template-Probe" bindet, so dass sich hier eine Y- bzw. T-förmige 3-Wege-Struktur ("three-way-junction") bildet.

US2004/023271 beschreibt ein Verfahren zum Nachweis von Analyten mit Hilfe eines zusammengesetzten Primers, welcher aus einem RNA-Bereich und aus einem 3' gelegenen DNA-Bereich besteht und mit dessen Hilfe in das Extensionsprodukt detektierbare Charakteristika eingefügt werden, die den Analyten identifizieren.

WO00/70095 beschreibt ein Verfahren, um viele Kopien einer Polynukleotid-Sequenz zu erzeugen. Dies wird mit Hilfe eines sogenannten Template-Switch Oligonukleotids erreicht, dessen 3' Bereich an die Zielsequenz bindet, nicht aber dessen 5'Bereich.

Mit Hilfe dieser beiden Verfahren werden kennzeichnende Nukleotidsequenzen während der Polymerisation in die neu synthetisierten Nukleinsäuren eingefügt. Diese beiden Verfahren haben den Nachteil, dass nicht bereits während der ersten Polymerase-Reaktion komplementäre Sequenzen in die neu synthetisierte Nukleinsäure eingeführt werden, so dass der neu syntehtisierte Nukleinsäurestrang nicht direkt Ausgangspunkt für unterschiedliche nachfolgende Reaktionen bilden kann.

Aufgabe der vorliegenden Erfindung ist es somit, ein neues Verfahren zur Einführung von Sequenzelementen (Tag-Sequenzen) in Nukleinsäuren anzugeben, welches die Mehrstufigkeit der oben beschriebenen bekannten Verfahren vermeidet und in einer ersten Polymerase-Reaktion ohne nachfolgende Gegenstrangsynthese am 3'-Ende der neu synthetisierten Nukleinsäure eine Tag-Sequenz einführt. Gelöst wird diese Aufgabe durch ein Verfahren zur Einführung mehrerer Tag-Sequenzen in eine Nukleinsäure, gekennzeichnet durch die folgenden Schritte gemäß Anspruch 1

Weitere vorteilhafte Ausgestaltungen, Aspekte und Details des erfindungsgemäßen Verfahrens ergeben sich aus den Patentansprüchen, der Beschreibung, den Beispielen sowie den Figuren.

Die vorliegende Offenbarung betrifft somit ein Verfahren zur Einführung von Sequenzelementen (einer Tag-Sequenz oder mehrerer Tag-Sequenzen) in eine Nukleinsäure, das durch die folgenden Schritte gekennzeichnet ist: (a) Bereitstellen einer Template-Nukleinsäure; (b) Hybridsieren mindestens einer Anker-Sequenz mindestens eines Anker-Oligonukleotids mit einem Sequenzabschnitt der Template-Nukleinsäure; und (c) Synthetisieren eines neuen Nukleinsäurestrangs, welcher partiell komplementär zur Template-Nukleinsäure ist und welcher an seinem 3'-Ende eine zu dem nicht-hybridisierenden Teil des Anker-Oligonukleotids (Tag-Template-Sequenz) komplementäre Sequenz enthält.

Die in dem erfindungsgemäßen Verfahren Verwendung findenden Anker-Oligonukleotide enthalten im Bereich ihres 5'-Endes mindestens eine spezifische Sequenz (Tag-Template-Sequenz), wodurch in den neu synthetisierten Strang eine zu dieser spezifischen (Tag-Template-) Sequenz komplementäre Sequenz (Tag-Sequenz) eingeführt wird. 3' dieser Tag-Template-Sequenz weist das Anker-Oligonukleotid mindestens eine zur Template-Nukleinsäure komplementäre Sequenz (Anker-Sequenz) auf, sodass eine Hybridisierung mit der Template-Nukleinsäure ermöglicht wird.

Neben den vorgenannten Schritten (a) bis (c) umfasst das erfindungsgemäße Verfahren auch noch einen Schritt (d), in dem der in Schritt (c) erzeugte Nukleinsäure-Doppelstrang weiter prozessiert wird. Diese Prozessierung kann naturgemäß einen oder eine Kombination aus mehreren Schritten umfassen. Während dieser weiteren Prozessierung können weitere Prozesse durchgeführt werden. Nur einige wenige Bespiele seien hier genannt:
1) Eine Trennung des Doppelstrangs in Einzelstränge. Vorzugsweise wird eine solche Strang-Trennung thermisch (d.h. durch Erwärmung), enzymatisch und/oder mittels chemischer Substanzen durchgeführt.
2) Eine Ligation von freien Enden von Nukleinsäuren durch natürlich vorkommende einzel- oder doppelstrangspezifische Ligasen oder andere Ligasen wie z.B. Ribozyme, chemische Ligation, beispielsweise mittels Thiophosphat (siehe US 6,635,425) oder Ligation mittels anderer zur Ligation befähigter Enzyme, beispielsweise Topoisomerasen.
3) Ein Prozessierung durch Nukleasen wie z.B. Endonukleasen oder Exonukleasen, die sequenzunspezifisch oder sequenzspezifisch Nukleinsäure schneiden/binden,
4) Eine RNA-Synthese mit bzw. ohne anschließende Translation der entstandenen RNA
5) Eine DNA-Synthese, z.B. Synthese eines Nukleinsäurestranges, welcher komplementär zu mindestens einem Teil des neu synthetisierten Nukleinsäurestrangs ist, oder z.b. die Amplifikation mindestens eines Teiles der Template-Nukleinsäure.
6) Eine Nukleinsäure-Nukleinsäure-Wechselwirkung, z.B. eine Wechselwirkung der neu synthetisierten Nukleinsäure mit Aptameren oder eine Hybridisierung der neu synthetisierten Nukleinsäure mit einer anderen Nukleinsäure, beispielsweise mit einem oder mehreren spezifischen Primern.
7) Eine Bindung eines oder mehrerer Proteine an die entstandenen Nukleinsäure-Stränge, z.B. zur, aber nicht eingeschränkt auf die, Aufreinigung oder Anreicherung bestimmter Nukleinsäure-Spezies mittels Protein-Bindung.
8) Eine Nukleinsäure-Markierung des neu-synthetisierten Nukleinsäurestrangs oder eines Amplifikationsproduktes des neu-synthetisierten Nukleinsäurestrangs.

Weitere Möglichkeiten der Prozessierung einer Doppelstrang-Nükleinsäure sind dem Fachmann geläufig. Die Art der Prozessierung ergibt sich zwingend aus den im individuellen Versuchsablauf erforderlichen Schritten und wird vom Fachmann entsprechend gewählt.

Die Erfindung offenbart somit ein Verfahren, durch das bei Nukleinsäure-Synthesen insbesondere am 3'-Ende ein Sequenzelement bzw. eine Tag-Sequenz in die neu synthetisierte Nukleinsäure eingeführt wird. Dabei wird ein Anker-Oligonukleotid mittels einer Anker-Sequenz an eine Ziel-Nukleinsäure bzw. Template-Nukleinsäure hybridisiert. Stößt die verwendete Polymerase während der Nukleinsäure-Synthese auf das Anker-Oligonukleotid, so erfolgt die weitere Synthese nicht entlang der Ziel-Nukleinsäure, sondern entlang des nicht-hybridisierenden Bereichs des Anker-Oligonukleotids (d.h. entlang der Tag-Template-Sequenz des Anker-Oligonukleotids), wodurch die Tag-Sequenz, eine Sequenz, die der Tag-Template-Sequenz komplementär ist, in die entstehende Nukleinsäure an deren 3'-Ende eingebaut wird. Bei dem erfindungsgemäßen Verfahren findet somit in Schritt (c) ein Template-Switch statt, d.h. die verwendete Polymerase springt von der Ziel- bzw. Template-Nukleinsäure auf das Anker-Oligonukleotid über. Im erfindungsgemäßen Verfahren finden somit Polymerasen Verwendung, die keine oder nur eine geringe Strand-Displacement-Aktivität aufweisen.

Das erfindungsgemäße Verfahren ermöglicht es erstmals, Tag-Sequenzen jeder beliebigen Sequenz bereits während der ersten Polymerase-Reaktion in vorher definierte oder zufällige Sequenzbereiche (die Anker-Sequenz des Anker-Oligonukleotids kann eine zufällige, degenerierte oder spezifische Sequenz sein) in eine Nukleinsäure einzuführen. Dadurch werden die oben diskutierten, aus dem Stand der Technik bekannten Verfahren dahingehend verbessert, dass für einen Einbau einer Tag-Sequenz in eine Nukleinsäure keine zusätzlichen Schritte notwendig sind, was Zeit- und Kosten-sparend ist.

Das erfindungsgemäße Verfahren kann durchgeführt werden, indem jeder der vorgenannten Schritte (a) bis (d) nacheinander jeweils in einem separaten Reaktionsgefäß erfolgt. In einer weiteren Ausführungsform werden die Schritte (a) bis (d) in ihrer Gesamtheit oder zwei oder mehr der genannten Schritte in einem einzigen Reaktionsgefäß durchgeführt werden, wodurch eine zusätzliche Zeitersparnis erreicht werden kann.

Das Sequenzelement (die Tag-Sequenz) wird insbesondere am 3'-Ende des neu synthetisierten Nukleinsäurestrangs eingeführt.

Als Nukleinsäure wird bei dem erfindungsgemäßen Verfahren insbesondere Desoxyribonukleinsäure (DNA), Ribonukleinsäure (RNA), Peptidnukleinsäure (PNA) oder eine Mischung dieser Nukleinsäuren eingesetzt. Nukleinsäuren können auch Basenanaloga enthalten, solange das erfindungsgemäße Verfahren hierdurch nicht beeinträchtigt wird.

Das bei dem erfindungsgemäßen Verfahren verwendete Anker-Oligonukleotid weist vorzugsweise mindestens eine 3' gelegene Ankersequenz und eine oder mehrere 5' zur Anker-Sequenz gelegene-Tag-Template-Sequenz(en) auf.

Die in dem Anker-Oligonukleotid enthaltene, mindestens eine Tag-Template-Sequenz wiederum enthält vorzugsweise mindestens eine funktionelle Sequenz.

Die Anker-Sequenz des erfindungsgemäß eingesetzten Anker-Oligonukleotids kann spezifisch, degeneriert oder zufällig ("random") sein. Die Anker-Sequenz des erfindungsgemäß eingesetzten Anker-Oligonukleotids enthält vorzugsweise mindestens 4 Basen, um eine ausreichende Hybridisierung zu gewährleisten. Insbesondere kann die Zahl der Basen bei etwa 4 bis etwa 50, vorzugsweise bei 4 bis 30, und besonders bevorzugt bei 6 bis 20 Basen liegen.

Die Synthese des neuen Nukleinsäurestrangs in Schritt (c) des erfindungsgemäßen Verfahren erfolgt vorzugsweise mittels einer Polymerase, die nur eine geringe und im günstigsten Fall keine Strand-Displacement-Aktivität zeigt ("Strand-Displacement" bezeichnet die Fähigkeit einer Polymerase, einen Nukleinsäure-Doppelstrang in seine Einzelstränge aufzuspalten; siehe auch unten). Das Template-Switch in dem erfindungsgemäßen Verfahren kann grundsätzlich umso effizienter erfolgen, je geringer die Strand-Displacement-Aktivität der eingesetzten Polymerase ist. Bestimmte spezifische Ausführungsformen des erfindungsgemäßen Verfahrens können jedoch durch eine geringe Strand-Displacement-Aktivität der Polymerase effektiver gestaltet werden (sie unten).

Das erfindungsgemäße Verfahren kann im Rahmen einer RNA-Amplifikation, einer Signal-Amplifikation mittels des so genannten "Rolling-Circle"-Verfahrens, einer DNA-Amplifikation, einer DNA-Amplifikation von methylierten DNA-Abschnitten, eines Verfahrens zur Einführung einer Schnittstelle für Restriktionsendonukleasen zur DNA-Klonierung, eines Verfahrens zur Einführung einer Schnittstelle für Restriktionsendonukleasen zur Herstellung zirkulärer DNA-Moleküle, eines Verfahrens zur Einführung einer Schnittstelle für Restriktionsendonukleasen zur nachfolgenden Ligation zu linearen Groß-Molekülen, eines Verfahrens für die Detektion von Nukleinsäure, beispielsweise in der PCR bzw. RT(Reverse Transkriptase)-PCR wie auch Real-Time PCR bzw. Real-Time RT-PCR (PCR = "Polymerase Chain Reaction", Polymerasekettenreaktion), eines Verfahrens für die Detektion und/oder Quantifizierung einer spezifischen Nukleinsäure, beispielsweise in der PCR bzw. RT-PCR wie auch Real-Time PCR bzw. Real-Time RT-PCR, eines Verfahrens zur Fusion von Transkriptom- oder Genomfragmenten, oder eines Verfahrens zur Fusion von Transkriptom- oder Genomfragmenten bei der Gesamt-Genom-Amplifikation ("Whole Genome Amplification") oder der Gesamt-Transkriptom-Amplifikation ("Whole Transcriptome Amplification") genutzt werden.

Nachfolgend werden einige häufiger verwendete Begriffe näher erläutert.

Polymerase: Polymerasen sind Enzyme, welche die Bildung von Phosphodiester-Bindungen zwischen einzelnen Nukleotiden innerhalb eines Nukleinsäurestrangs katalysieren (z.B. DNA- und RNA-Polymerasen). Besonders bevorzugt sind für den Einsatz bei dem erfindungsgemäßen Verfahren in Schritt (c) alle Polymerasen, die bei den gewählten Versuchsbedingungen eine geringe oder keine Strand-Displacement-Aktivität zeigen. Im Falle einer zu hohen Strand-Displacement-Aktivität kann es dazu kommen, dass das Anker-Oligonukleotid verdrängt wird, was den Einbau der Tag-Sequenz behindern und im Extremfall ganz verhindern könnte. Ganz besonders bevorzugt werden daher bei dem erfindungsgemäßen Verfahren bei dem Polymerisierungsschritt Polymerasen eingesetzt, die kein Strand-Displacement zeigen. Zu diesen bevorzugten Polymerasen gehören neben anderen Pölymerasen mit nur geringer Strand-Displacement-Aktivität auch das Klenow-Fragment der DNA-Polymerase I, T4-DNA-Polymerase, T7-DNA-Polymerase, die DNA-Polymerase I, oder auch DNA- und RNA-abhängige Reverse Transkriptasen. Zu letzteren gehören Enzyme aus Viren, Bakterien, Archae-Bakterien und Eukaryonten. Hierzu zählen z.B. auch Enzyme aus Introns, Retrotransposons oder Retroviren wie z.B. MMLV, AMV, HIV etc. Aber auch andere Polymerasen, welche nur in geringfügigem Maße eine Strand-Displacement-Aktivität aufweisen, sind geeignet, mittels des erfindungsgemäßen Verfahrens eine Tag-Sequenz in einen entstehenden Nukleinsäurestrang einzubauen. Bestimmte spezifische Ausführungsformen des erfindungsgemäßen Verfahrens können durch Polymerasen mit einer geringen Strand-Displacement-Aktivität effektiver gestaltet werden (siehe unten). Unter einer geringen Strand-Displacement Aktivität im Sinne der vorliegenden Erfindung wird eine Wahrscheinlichkeit des Strand-Displacement von weniger als 30 %, vorzugsweise weniger als 50 % und besonders bevorzugt weniger als 80 % der Anker-Oligonukleotide durch die Polymerase verstanden. Dem Fachmann ist bekannt, dass sich die Strand-Displacement-Wahrscheinlichkeit in Abhängigkeit von der Reaktionstemperatur, den Pufferbedingungen, der jeweiligen Polymerase und des hybridisierenden Anteils des Anker-Oligonukleotids verändern kann.

In oben genanntem Sinne für den Einsatz in Schritt (c) des erfindungsgemäßen Verfahrens geeignete Polymerasen sind z.B. hitzelabile Polymerasen. Grundsätzlich eignen sich alle hitzelabilen Polymerasen, die bei den gewählten Versuchsbedingungen eine geringe oder keine Strand-Displacement-Aktivität zeigen. Zu diesen hitzelabilen Polymerasen gehören beispielsweise DNA-Polymerasen, die eine Reparatur der zelleigenen DNA durchführen (so genannte Repair-Polymerasen), aber auch Replikasen. Da Replikasen häufig eine ausgeprägte Strand-Displacement Aktivität aufweisen, sollten Replikasen verwendet werden, deren Strand-Displacement Aktivität klein ist oder durch Mutation (z.B. Lys143-Mutation der Phi29-DNA-Polymerase, de Vega et al., An invariant lysine residue is involved in catalysis at the 3'-5' exonuclease active site of eukaryotic-type DNA polymerases; J. Mol. Biol.; 270(1):65-78, 1997), Modifikation oder Wegfall von akzessorischen Faktoren reduziert oder eliminiert wurde. Zu diesen hitzelabilen Polymerasen gehören weiterhin beispielsweise DNA-Polymerasen wie das Klenow-Fragment der DNA-Polymerase I, die DNA-Polymerase I, die T4-Polymerase, die T7-Polymerase oder auch Reverse Transkriptasen. Im Falle der vorliegenden Erfindung wird eine Polymerase dann als hitzelabil bezeichnet, wenn diese nach einer Behandlung von 10 Minuten bei einer Temperatur von 65°C nur noch eine Aktivität von maximal 20 % der Ausgangsaktivität aufweist, d.h. wenn die Polymerase zu mindestens 80 % inaktiviert worden ist

Neben hitzelabilen Polymerasen kann aber auch jede nicht-hitzelabile Polymerase in Schritt (c) des erfindungsgemäßen Verfahrens verwendet werden. Grundsätzlich eignen sich alle nicht-hitzelabilen Polymerasen, die bei den gewählten Versuchsbedingungen eine geringe oder keine Strand-Displacement-Aktivität zeigen. Entsprechende Polymerasen sind kommerziell erhältlich und dem Fachmann bekannt. Zu diesen nicht-hitzelabilen Polymerasen gehören beispielsweise DNA-Polymerasen, die eine Reparatur der zelleigenen DNA durchführen (so genannte Repair-Polymerasen), aber auch Replikasen. Da Replikasen häufig eine ausgeprägte Strand-Displacement Aktivität aufweisen, sollten Replikasen verwendet werden, deren Strand-Displacement Aktivität klein ist oder durch Mutation, Modifikation oder Wegfall von akzessorischen Faktoren reduziert oder eliminiert wurde. Als Beispiele für nicht-hitzelabile Polymerasen sei an dieser Stelle die Taq-Polymerase, das Klenow-Fragment der Taq-Polymerase und Pfu-Polymerase, bevorzugt ohne Exonuklease-Aktivität (Pfu exo-), genannt.

**DNA-Polymerase mit Strand-Displacement-Aktivität:** Strand-Displacement-Aktivität einer DNA-Polymerase bedeutet, dass das eingesetzte Enzym dazu in der Lage ist, einen DNA-Doppelstrang in zwei Eihzelstränge aufzutrennen. DNA-Polymerasen mit Strand-Displacement-Aktivität, die beispielsweise bei der RCA eingesetzt werden können, sind z.B. Holoenzyme oder Teile von Replikasen aus Viren, Prokaryonten, Eukaryonten, oder Archaeen, Phi 29-artige DNA-Polymerasen" die DNA-Polymerase Klenow exo⁻ und die DNA-Polymerase aus Bacillus stearothermophilus mit der Bezeichnung Bst exo⁻. "exo⁻" gedeutet, dass das entsprechende Enzym keine 5'-3'-Exonukleaseaktivität aufweist. Ein bekannter Vertreter der Phi 29-artigen DNA-Polymerasen ist die DNA-Polymerase aus dem Bakteriophagen Phi 29. Andere Phi 29-artige DNA-Polymerasen kommen z.B. in den Phagen Cp-1, PRD-1, Phi 15, Phi 21, PZE, PZA, Nf, M2Y, B103, SF5, GA-1, Cp-5, Cp-7, PR4, PR5, PR722 und L 17 vor. Weitere geeignete DNA-Polymerasen mit Strand-Displacement-Aktivität sind dem Fachmann bekannt. Alternativ werden unter DNA-Polymerasen mit Strand-Displacement-Aktivität auch solche DNA-Polymerasen ohne Strand-Displacement-Aktivität verstanden, wenn neben einer entsprechenden DNA-Polymerase einen Katalysator verwendet wird, beispielsweise ein Protein oder ein Ribozym, welcher die Trennung eines DNA-Doppelstrangs oder die Stabilisierung von DNA-Einzelsträngen ermöglicht. Zu diesen Proteinen gehören z.B. die Helicasen, SSB-Proteine und Rekombinationsproteine, die als Bestandteil größerer Enzymkomplexe wie z.B. Replikasen enthalten sein können, In diesem Falle erzeugt man mit Komponenten zusätzlich zu der Polymerase eine Polymerase mit Strand-Displacement-Aktivität. Die Polymerasen mit Strand-Displacement Aktivität können hitzelabil oder hitzestabil sein.

**Template-Nukleinsäure**: Als Template-Nukleinsäure kommen insbesondere Desoxyribonukleinsäure (DNA), Ribonukleinsäure (RNA), Peptid-Nukleinsäure (PNA) oder eine Mischung dieser Nukleinsäuren in Frage. Die Template-Nukleinsäure kann auch in einer chemisch modifizierten Form vorliegen. Sie kann Basenanaloga (z.B. auch non-Purin- oder non-Pyrimidin-Analoga) enthalten oder aus diesen bestehen, soweit eine Hybridisierung mit einem Partner-Strang erfolgen kann. Ferner kann die Template-Nukleinsäure auch andere Modifikation enthalten, wie. z.B. Fluorophor(e), Biotin, Methylierung(en) etc. Entsprechende alternative Modifikationen sind dem Fachmann hinlänglich bekannt. Notwendige Eigenschaften der Template-Nukleinsäure sind, dass an diese ein Anker-Oligonukleotid hybridisieren kann und diese als Ziel von einer Polymerase erkannt werden kann. Die Template-Nukleinsäure kann unterschiedlicher Länge sein und einzeisträngig, partiell doppelsträngig oder doppelsträngig sein. Im Falle einer doppelsträngigen Template-Nukleinsäure muss zunächst deren Denaturierung erfolgen um eine Hybridisierung zumindest der Anker-Sequenz des Anker-Oligonukleotids zu ermöglichen.

**Anker-Oligonukleotid:** Die Anker-Oligonukleotide enthalten im Bereich ihres 5'-Endes mindestens eine spezifische Sequenz-(Tag-Template-Sequenz), wodurch in den neu synthetisierten Strang eine zu dieser spezifischen (Tag-Template-) Sequenz komplementäre Sequenz (Tag-Sequenz) eingeführt wird. 3' dieser Tag-Template-Sequenz weist das Anker-Oligonukleotid mindestens eine zur Template-Nukleinsäure komplementäre Sequenz (Anker-Sequenz) auf, sodass eine Hybridisierung mit der Template-Nukleinsäure ermöglicht wird. Zudem kann (können) zwischen der 3'-gelegenen Anker-Sequenz und der 5'-gelegenen Tag-Template-Sequenz noch eine oder mehrere weitere Sequenz(en) eingeschoben sein, die eine oder mehrere andere Funktionen übernehmen kann (können), wie z.B. Sequenzen für Primer-Bindestellen, Sonden-Bindestellen, Promotoren zur Initiation der Transkription, Restriktionsendonuklease-Schnittstellen, und Ribosomen-Bindestellen etc. Beliebige Kombinationen der vorgenannten Funktionen in der weiteren Sequenz sind möglich.

Anker-Sequenz: Die Anker-Sequenz des Anker-Oligonukleotids kann an der zur Anker-Sequenz komplementären Sequenz der Template-Nukleinsäure hybridisieren. Die Anker-Sequenz kann eine Zufallssequenz, eine degenerierte Sequenz oder eine spezifische Sequenz enthalten. Diese Anker-Sequenz kann von unterschiedlicher Länge sein. So kann z.B. eine spezifische Anker-Sequenz eine Sequenz enthalten, die z.B. an der Sequenz eines Transkripts, beispielsweise an oder teilweise an der Sequenz des Poly-A Tails von Poly-A-RNA, oder eines Gens spezifisch hybridisieren kann. Des Weiteren kann die Anker-Sequenz auch funktionelle Sequenzen enthalten. Die Anker-Sequenz kann ihrer Natur nach RNA, DNA oder eine Mischung aus beiden sein. Sie kann Basenanaloga (z.B. auch non-Purin- oder non-Pyrimidin-Analoga) oder Nukleotidanaloga (z.B. PNA) enthalten oder aus diesen bestehen, soweit eine Hybridisierung mit der Ziel-Nukleinsäure erfolgen kann. Ferner kann die Anker-Sequenz auch *Minor-Groove-Binder* enthalten. Schließlich kann die Anker-Sequenz andere Modifikation enthalten, wie. z.B. Fluorophor(e), Biotin, Methylierung(en) etc. Entsprechende alternative Modifikationen sind dem Fachmann hinlänglich bekannt.

**Tag-Template-Sequenz und Tag-Sequenz:** Die Tag-Template-Sequenz des Anker-Oligonukleotids ist die Sequenz, die als Matrize für die Tag-Sequenz dient, welche in den neu synthetisierten Nukleinsäure-Strang eingebaut wird. Die Tag-Template-Sequenz kann eine bestimmte Sequenz enthalten. Die Tag-Template-Sequenz kann aber auch eine degenerierte Sequenz enthalten, sofern diese nicht oder nur schlecht an der Ziel-Nukleinsäure hybridisiert. Diese Tag-Sequenz kann unterschiedlicher Länge sein. Die Tag-Sequenz kann neben der Tatsache, dass sie in den zu synthetisierenden Nukleinsäure-Strang eingebaut wird, noch weitere funktionelle Sequenzen enthalten, so dass z.B. Hybridisierungsstelle, Primer-Bindestellen, Sonden-Bindestellen, Promotoren, Signalsequenzen zur Initiation der Transkription und/oder Translation, Restriktionsendonuklease-Erkennungs- und Schnitt-Stellen, Ribosomen-Bindestellen, Protein-Bindestelle, Antikörper-Erkennungsstelle, etc. oder deren komplementäre Sequenzen in den zu synthetisierenden Nukleinsäurestrang eingeführt werden. Außerdem kann die Tag-Sequenz auch Kombinationen dieser funktionellen. Sequenzen enthalten. Die Tag-Template-Sequenz kann ihrer Natur nach RNA, DNA oder eine Mischung aus beiden sein. Die Tag-Template-Sequenz sowie die Tag-Sequenz können Basenanaloga (z.B. auch non-Purin oder non-Pyrimidin-Analoga) oder Nukleotidanaloga (z.B. PNA) enthalten oder aus diesen bestehen. Die Tag-Template-Sequenz sowie die Tag-Sequenz können weitere Modifikation enthalten wie z.B. Fluorophor(e), Biotin, Methylierung(en) etc. Entsprechende Ausführungsformen/Modifikationen sind dem Fachmann bekannt.

**3'-Ende des Anker-Oligonukleotids:** Das 3'-Ende des Anker-Oligonukleotids kann eine freie 3'-OH Gruppe tragen, die freie 3'-OH-Gruppe kann alternativ jedoch auch blockiert sein, das heißt, das Anker-Oligonukleotid ist beispielsweise an seinem 3'-Ende nicht mehr verlängerbar, beispielsweise durch eine Polymerase. Alternativ kann am 3'-Ende des Anker-Oligonukleotids auch ein Dideoxy-Nukleotid eingebaut werden, so dass auch in diesem Falle das Anker-Oligonukleotid nicht verlängert werden kann, beispielsweise durch eine Polymerase. Das 3'-Ende des Anker-Oligonukleotids kann für bestimmte Applikationen Modifikationen öder Anhänge tragen. Solche Anhänge bzw. Modifikationen können z.B. Fluorophor(e), Biotin, Methylierung(en) etc. Entsprechende Ausführungsformen/Modifikationen sind dem Fachmann bekannt. In Ausführungsformen der vorliegenden Erfindung, in denen das Anker-Oligonukleotid gleichzeitig als Primer fungiert, muss das 3'-Ende des Anker-Oligonukleotids durch eine Polymerase verlängerbar sein, vorzugsweise ein 3'-OH-Ende aufweisen.

**5'-Ende des Anker-Oligonukleotids:** Das 5'-Ende des Anker-Oligonukleotids kann eine freie Phosphat-Gruppe aufweisen, die freie Phosphat-Gruppe kann jedoch alternativ auch blockiert sein oder fehlen. Das 5'-Ende des Anker-Oligonukleotids kann für bestimmte Applikationen Modifikationen oder Anhänge tragen. Solche Anhänge bzw. Modifikationen können z.B. Fluorophor(e), Biotin, Methylierung(en) etc. Entsprechende Ausführungsformen/Modifikationen sind dem Fachmann bekannt.

**Rolling Circle Amplifikation (RCA):** Die Rolling Circle Amplifikation ist auch unter der Bezeichnung "Rolling Circle Replikation" bekannt. Bei der RCA hybridisiert mindestens ein Primer an der zirkulären Ziel-Sequenz. Durch Verwendung einer DNA-Polymerase mit Strand-Displacement-Aktivität wird der Primer verlängert und kann durch fortlaufende Synthese an der zirkulären Ziel-Sequenz über die Primer-Bindestelle hinaus zu einem konkatemeren Molekül polymerisiert werden. Dabei kann der verwendete Primer eine zufällige, degenerierte oder spezifische Sequenz aufweisen. Der Primer kann dabei aus DNA, RNA, PNA oder modifizierten Basen oder Basenanaloga bestehen, sofern eine Hybridisierung mit der Ziel-Sequenz und eine Verlängerung des Primers durch eine Polymerase erfolgen kann. Der Primer kann auch durch einen Einzelstrang-Bruch in der zumindest partiell doppelsträngigen zirkulären Ziel-Sequenz entstehen. Alternativ dazu können auch DNA-Polymerasen ohne Strand-Displacement-Aktivität verwendet werden, in diesem Fall muss jedoch ein Enzym-Cocktail eingesetzt werden, der neben einer entsprechenden DNA-Polymerase einen Katalysator, beispielsweise ein Protein oder ein Ribozym, enthält, welcher die Trennung eines DNA-Doppelstrangs oder die Stabilisierung von DNA-Einzelsträngen ermöglicht. Zu diesen Proteinen gehören z.B. die Helicasen, SSB-Proteine und Rekombinationsproteine.

**Amplifikation:** Unter einer Amplifikation einer Nukleinsäure wird die Vermehrung des Templates um mindestens den Faktor 2 oder mehr verstanden. Hierzu kann die Nukleinsäure linear oder exponentiell vermehrt werden. Eine lineare Amplifikation erreicht man beispielsweise mittels RCA in Gegenwart von Primern, die auf dem Target Circle nur mit einer spezifischen Sequenz hybridisieren. Eine exponentielle Amplifikation erreicht man beispielsweise über RCA mit Primern, wobei die Primer mit mindestens 2 Bindestellen auf dem Target Circle hybridisieren oder aber mit mindestens einer Bindestelle auf dem Target Circle und mindestens einer Bindestelle auf dem komplementären Strang hybridisieren. Weitere lineare und exponentielle für die vorliegende Erfindung geeignete Amplifikationsverfahren sind dem Fachmann geläufig, beispielsweise PCR, NASBA, SDA, MDA, TMA, 3SR, usw.

**Primer:** Unter Primer im Sinne der vorliegenden Erfindung wird ein Molekül verstanden, das als Startstelle für ein Enzym mit Nukleinsäure-Polymerase-Aktivität dient. Dieser Primer kann ein Protein, eine Nukleinsäure oder ein anderes Molekül sein, das sich dem Fachmann als Polymerase-Startstelle geeignet erweist. Dieses Molekül kann durch intermolekulare aber auch durch intramolekulare Wechselwirkung als Startstelle dienen. Im Falle von Nukleinsäure-Primem müssen diese nicht, können aber über ihre gesamte Länge mit der Template-Nukleinsäure hybridisieren.

**Erststrang-Synthese:** Unter einer Erststrang-Synthese wird im Rahmen der vorliegenden Erfindung die Polymerase-abhängige Nukleinsäuresynthese verstanden, bei der der neu synthetisierte Nukleinsäurestrang komplementär zu einer Template-Nukleinsäure polymerisiert wird und wobei am 3'-Ende des neu synthetisierten Nukleinsäurestrangs während der Erststrang-Synthese Matrizenabhängig eine Tag-Sequenz eingeführt wird. Bei der Einführung der Tag-Sequenz findet keinerlei 'Tailing' statt, das heißt, es werden in dem der vorliegenden Erfindung zu Grunde liegenden Verfahren keinerlei Nukleotide Matrizen-unabhängig in die neu synthetisierte Nukleinsäure eingeführt. Die Matrize zur Einführung der Tag-Sequenz am 3'-Ende des neu synthetisierten Nukleinsäurestrangs ist vorzugsweise die Tag-Template-Sequenz des Anker-Oligonukleotids, welches während der Erststrang-Synthese mittels seiner Anker-Sequenz an der Template-Nukleinsäure hybridisiert ist.

Eine Ausführungsform des erfindungsgemäßen Verfahrens betrifft eine selektive Amplifikation von methylierten bzw. nicht methylierten DNA-Abschnitten.

Bei der selektiven Amplifikation von methylierten bzw. nicht methylierten DNA-Abschnitten wird während einer Nukleinsäure-Synthesereaktion ausgehend von DNA mittels der Tag-Template-Sequenz des Anker-Oligonukleotids eine Tag-Sequenz am 3'-Ende der neu synthetisierten Nukleinsäure eingeführt. Bevorzugt findet in Schritt (c) mindestens ein Primer Verwendung, der eine Tag-Sequenz aufweist. Diese ist vorzugsweise zumindest partiell identisch zur Tag-Template-Sequenz des Anker-Oligonukleotids. Partiell identisch im Sinne der vorliegenden Erfindung bedeutet, dass mindestens so viele Nukleotide identisch sind, dass der neu synthetisierte Nukleinsäurestrang an dieser Stelle einen unter den jeweiligen Versuchsbedingungen stabilen Doppelstrang bildet. Hierfür ist erfahrungsgemäß eine Sequenz mit einer Länge von mindestens ca. 6 Nukleotiden notwendig.

Bei der selektiven Amplifikation von methylierten bzw. nicht methylierten DNA-Abschnitten wird nach der Nukleinsäure-Synthesereaktion in Schritt (c) die DNA mittels methylierungssensitiver Restriktionsendonukleasen geschnitten. Methylierte Sites werden von diesen Restriktionsendonukleasen nicht erkannt und entsprechend nicht geschnitten. Schneiden die methylierungssensitiven Restnktionsendonukleasen hemi-methylierte DNA, so muss der Schritt (c) des erfindungsgemäßen Verfahrens in Anwesenheit von methylierten dNTPs, bevorzugt methyliertes dCTP, durchgeführt werden. Als methylierungssensitve Restriktionsendonukleasen können beispielsweise folgende Enzyme eingesetzt werden: Hpa II, AatII, BcnI, Bsh12361, Bsh1285I, BshTI, Bsp681, Bsp119II, Bsp143II, Bsu15II, CseI, Cfr10I, Cfr421, Cpol, Eco47III, Eco52I, Eco72I, Eco88I, Eco105I, EheI, Esp3I, FspAI, Hhal, Hin1I, Hin6I, HpalI, Kpn2I, MbiI, MluI, NotI, NsbI, PauI, PdiI, Pfl23II, Psp14061, Pvul, SalI, Sgsl, SmaI, Smul, Ssil, TaiI, Taul, XhoI, etc.

Bei der selektiven Amplifikation von methylierten bzw. nicht methylierten DNA-Abschnitten kann die DNA alternativ mittels methylierungsspezifischer Restriktionsendonukleasen geschnitten werden. Bei Verwendung solcher Endonukleasen werden entsprechend nachfolgend die nicht-methylierten DNA-Abschnitte selektiv amplifiziert. Hiernach wird der neu synthetisierte Nukleinsäurestrang vom Template-Strang getrennt. Die Strangtrennung kann dabei thermisch, enzymatisch und/oder chemisch erfolgen. In den nicht geschnitten Abschnitten der DNA, also den methylierten Bereichen, können sich nach der Strangtrennung intramolekulare Haamadelstrukturen ausbilden. Diese können wie bereits oben beschrieben für Ligationsreaktionen genutzt werden, um die Nukleinsäuren in einer nachfolgenden Reaktion zu amplifizieren, beispielsweise mittels Rolling Circle Amplification.

Eine bevorzugte Ausführungsform des erfindungsgemäßen Verfahrens betrifft die Einführung einer Tag-Sequenz zur Detektion von Template-Nukleinsäuren.

Die Template-Nukleinsäure gemäß dieser Ausführungsform des erfindungsgemäßen Verfahrens kann z.B. DNA oder RNA sein. Vorzugsweise wird für die Detektion der Template-Nukteinsäure eine Amplifikation einer Nukleinsäure durchgeführt. Bei dieser dritten Ausführungsform des erfindungsgemäßen Verfahrens können zwei grundlegende Aspekte unterschieden werden. Zum einen kann die in Schritt (c) neu synthetisierte Nukleinsäure selbst amplifiziert werden, zum anderen kann die in Schritt (c) neu synthetisierte Nukleinsäure als Primer bei der Amplifikation einer Detektions-Nukleinsäure dienen.

Wird die in Schritt (c) neu synthetisierte Nukleinsäure zum Zwecke der Detektion ampliftziert, so enthält in einer ersten Ausführungsform die Tag-Template-Sequenz des eingesetzten Anker-Oligonukleotids mindestens eine Sequenz, die komplementär zu einer Primer-Bindestelle ist. In diesem Fall erfolgt in der Regel in einem weiteren Schritt (d) eine Prozessierung des gebildeten Nukleinsäurekonstrukts, wobei zunächst eine Strangtrennung erfolgt, die vorzugsweise thermisch, enzymatisch und/oder chemisch erfolgt Im Anschluss an die Strangtrennung erfolgt eine Amplifikation des neu gebildeten Nukleinsäurestrangs. Die Amplifikation des neu synthetisierten Nukleinsäurestrangs erfolgt In Gegenwart mindestens eines Primers, wobei dessen Primer-Bindestelle über die Tag-Template-Sequenz des eingesetzten Anker-Oligonukleotids in den neu synthetisierten Nukleinsäurestrang eingebracht wird. Mit Hilfe eines einzelnen Primers kann die neu synthetisierte Nukleinsäure beispielsweise mittels eines linearen Amplikationsverfahrens amplifiziert werden. In einer weiteren bevorzugten Ausführungsform kann mindestens ein weiterer zur Zielnukleinsäure komplementärer Primer zugesetzt werden, sodass eine exponentielle Amplifikation der neu synthetisierten Nukleinsäure ermöglicht wird. Der mindestens eine bei der Amplifikation des neu synthetisierten Stranges Verwendung findende Primer kann eine Nukleinsäure oder ein Peptid bzw. Protein sein.

In einer weiteren bevorzugten Ausführungsform wird Schritt (c) der Ausführungsform des erfindungsgemäßen Verfahrens, d.h. während der Polymerisation des neu synthetisierten Nukleinsäurestrangs, in Gegenwart mindestens eines Primers durchgeführtder in seinem 5'-Bereich eine Tag-Sequenz aufweist. Dieser Primer mit Tag-Sequenz bindet die Zielnukleinsäure mit seinem 3'-Bereich erfindungsgemäß in einem Bereich, der 3' von der Bindestelle des Anker-Oligonukleotids an der Zielnukleinsäure liegt. Der 3'-Bereich des Primers mit Tag-Sequenz kann spezifisch, degeneriert oder zufällig sein. Gemäß einem bevorzugten Aspekt enthält die Tag-Sequenz des Primers eine Sequenz, die komplementär zu einer Primer-Bindestelle ist. In weiteren bevorzugten Ausführungsformen kann die Tag-Sequenz auch andere und/oder weitere funktionelle Sequenzen enthalten. Die Sequenzen, die in der Tag-Template-Sequenz des Anker-Oligonukleotids und in der Tag-Sequenz des Primers aus Schritt (c) enthalten und komplementär zu den Primer-Bindestellen sind, können identisch oder verschieden sein. Mit Hilfe eines einzelnen Primers kann die neu synthetisierte Nukleinsäure beispielsweise mittels eines linearen Amplifikationsverfahrens amplifiziert werden. In einer weiteren bevorzugten Ausführungsform kann mindestens ein weiterer Primer zugesetzt werden, sodass eine exponentielle Amplifikation der neu synthetisierten Nukleinsäure ermöglicht wird. Dieser mindestens eine weitere Primer kann a) zur Zielnukleinsäure komplementär sein oder b) mit der Tag-Sequenz des Primers identisch sein oder c) vollständig oder teilweise identisch mit dem Teil des Primers mit Tag-Sequenz aus Schritt (c) sein, der an die Zielnukleinsäure bindet oder d) die Fusionsstelle des Primers mit Tag-Sequenz aus Schritt (c) zwischen dem zur Zielnukleinsäure komplementären Bereich und Tag-Sequenz umfassen.

Die bei den beschriebenen Ausführungsformen des erfindungsgemäßen Verfahrens Verwendung findenden Amplifikationverfahren können beispielsweise Polymerase-Kettenreaktion (Polymerase Chain Reaction, "PCR") Strand Displacement Amplfication (SDA), Nucleic Acid Sequence Based Amplification (NASBA), Seff Sustained Sequence Amplification (3SR), Rolling Circle Amplification (RCA) oder Multiple Displacement Amplification (MDA) sein. Weitere im erfindungsgemäßen Verfahren einsetzbare Amplifikationsverfahren sind dem Fachmann geläufig.

Gemäß einer weiteren bevorzugten Ausführungsform des erfindungsgemäßen Verfahrens weisen der in Schritt (c) verwendete Primer eine Tag-Sequenz und das Anker-Oligonukleotid eine Tag-Template-Sequenz auf, die identisch oder zumindest partiell identisch sind.

Gemäß einer bevorzugten Ausführungsform des erfindungsgemäßen Verfahrens wird nach dem Schritt (c) in einem weiteren Schritt (d) eine Prozessierung vorgenommen, bei der alle neu entstandenen Doppelstrang-Enden ohne vorherige Strangtrennung ligiert werden. Nach der entsprechenden Ligation kann dann noch eine Strangtrennung durchgeführt werden, wobei die Strangtrennung vorzugsweise thermisch, enzymatisch und/oder chemisch erfolgt. Die Nukleinsäure-Synthese kann dann wiederum mittels Rolling Circle Ampliflcation durchgeführt werden.

Eine Detektion der gebildeten Nukleinsäure kann mittels dem Fachmann bekannter und geeignet erscheinender Verfahren erfolgen. Zu diesen an sich bekannten Methoden gehören z.B. die Fluoreszenz-Detektion mittels Fluoreszenzsonden oder Detektion mittels fluoreszierender Nukleinsäure-bindender Substanzen, beispielsweise SYBR Green^{®}, Ethidiumbromid, PicoGreen^{®}, RiboGreen^{®}**,** usw. Bei der Fluoreszenz-Detektion können auch beispielsweise die bei der Amplifikation eingesetzten Nukleotide mit einem Fluorophor markiert sein. Eine Detektion der Fluoreszenzsignale und/oder eine Größenbestimmung der erzeugten Nukleinsäureabschnitte kann z.B. durch eine Gelelektrophorese, eine Kapillarelektrophorese, Arrays, Fluoreszenzdetektions-Geräte oder Real Time-Cycler erfolgen. Die Detektion der Nukleinsäure kann auch mittels ihrer längenabhängigen Masse erfolgen. Grundsätzlich können alle erfindungsgemäßen Verfahren zur Amplifikation einer Nukleinsäure auch für die Detektion der amplifizierten Nukleinsäure Verwendung finden, wobei während oder nach der Amplifikation die Detektion erfolgt.

Gemäß eines weiteren bevorzugten Aspekts des erfindungsgemäßen Verfahrens enthält die Tag-Template-Sequenz des Anker-Oligonukleotids und/oder die Sequenz des Primers mit Tag-Sequenz aus Schritt (c) mindestens eine weitere Sequenz, die die Bindung einer Nukleinsäure-Sonde ermöglicht. An die so eingeführten Sonden-Bindestellen können zur Detektion geeignete Sonden gebunden werden. Dazu sind alle dem Fachmann im Zusammenhang mit der Detektion von Nukleinsäuren bekannten Sonden einsetzbar, beispielsweise mit einem Fluoreszenzfarbstoff assoziierte Sonden oder auch radioaktiv markierte Sonden.

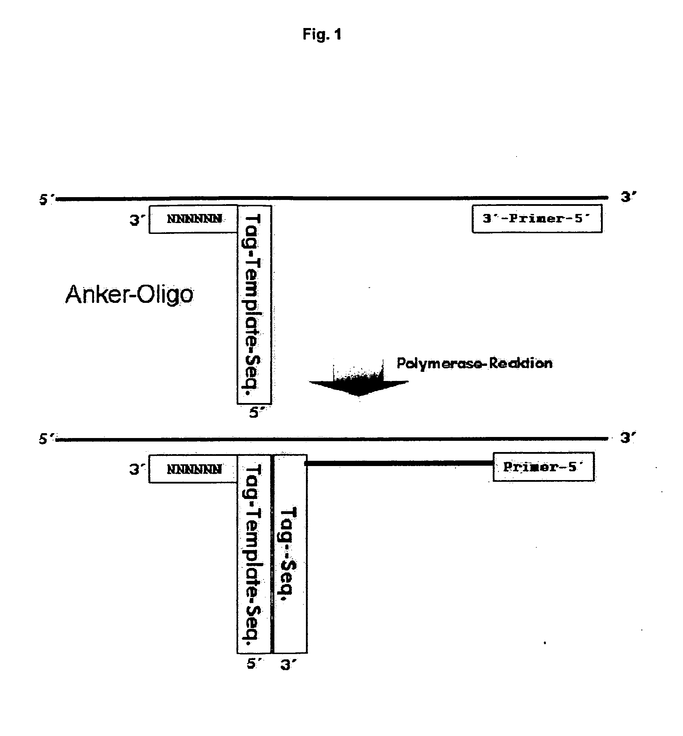

In der Zeichnung zeigt
- Fig. 1: schematisch den Einbau einer Tag-Sequenz mittels der Tag-Template-Sequenz eines Anker-Oligonukleotids während einer DNA-Synthese mittels einer Polymerase;

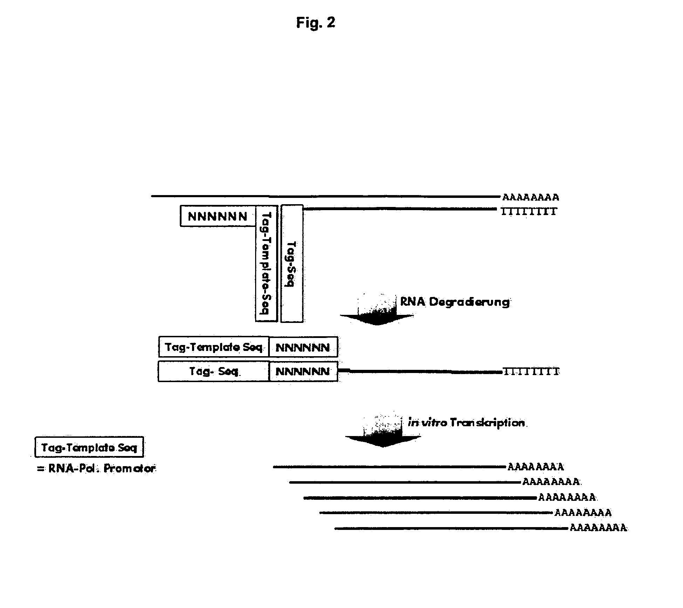

- Fig. 2: eine schematische Darstellung eines erfindungsgemäßen Verfahrens im Rahmen einer RNA-Amprifikation, bei der während der Synthese des neuen Nukleinsäurestranges eine Sequenz komplementär zur Promotor-Sequenz mittels einer Tag-Template-Sequenz eines Anker-Oligonukleotid in den neu synthetisierten Nukleinsäurestrang eingeführt wird;

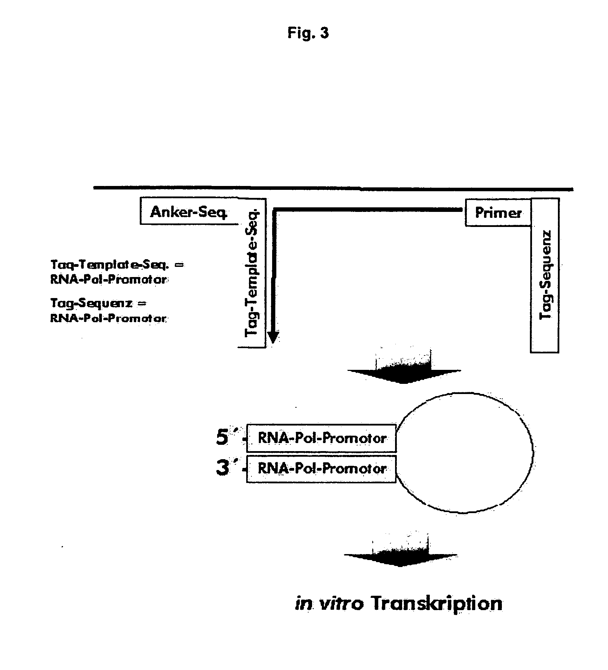

- Fig. 3: eine schematische Darstellung eines weiteren erfindungsgemäßen Verfahrens im Rahmen einer RNA-Amplifikation, bei der während der Synthese des neuen Nukleinsäurestranges eine Promotor-Sequenz eingebracht wird, indem die Tag-Sequenz des Primers enthaltend die Promotor-Sequenz und die durch die Tag-Template-Sequenz des Anker-Oligonukleotids enthaltend die Promotor-Sequenz eingebrachte Tag-Sequenz des neu synthetisierten Stranges miteinander hybridisieren und so den Promotor bilden;

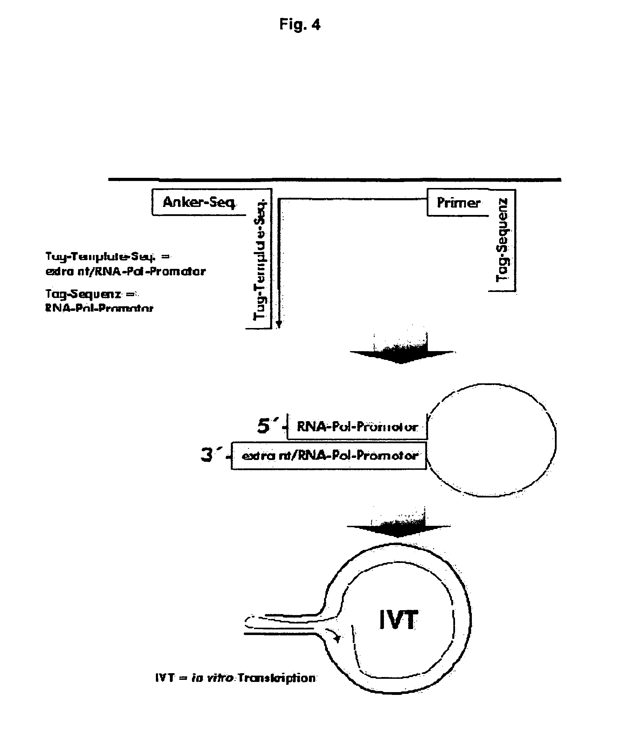

- Fig. 4: eine schematische Darstellung eines weiteren erfindungsgemäßen Verfahrens im Rahmen einer RNA-Amplifikation, bei der während der Synthese des neuen Nukleinsäurestranges eine Promotor-Sequenz eingebracht wird, indem die Tag-Sequenz des Primers enthaltend die Promotor-Sequenz und die durch Tag-Template-Sequenz des Anker-Oligonukleotids enthaltend die Promotor-Sequenz eingebrachte Tag-Sequenz miteinander hybridisieren und so den Promotor bilden, wobei die Tag-Template-Sequenz des Anker-Oligonukleotids 5' der Promotor-Sequenz mindestens ein weiteres Nukleotid (extra nt) aufweist;

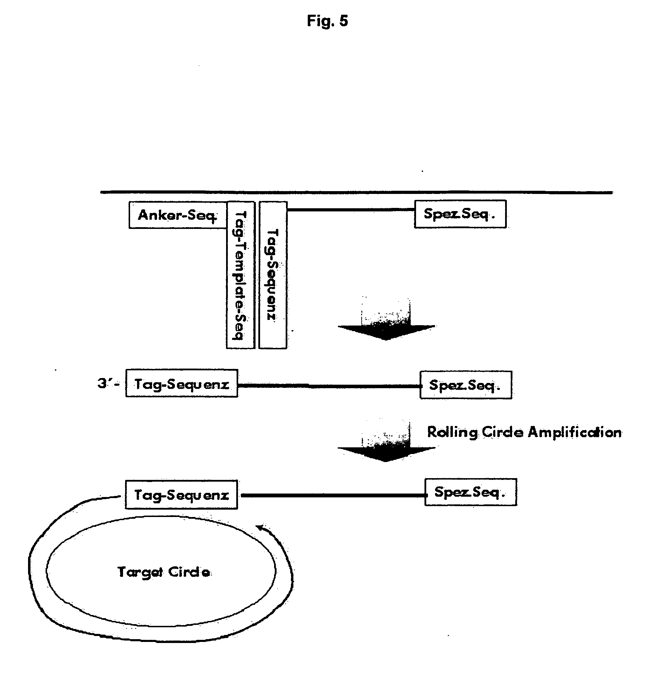

- Fig. 5: eine schematische Darstellung eines generisch anwendbaren, erfindungsgemäßen Verfahrens im Rahmen einer Signal-Amplifikation, bei der während der Synthese des neuen Nukleinsäurestranges mittels der Tag-Template-Sequenz des Anker-Oligonukleotids eine Tag-Sequenz in den neu synthetisierten Strang eingebracht wird, wobei der neu synthetisierte Strang an seinem 3'-Ende mittels seiner Tag-Sequenz einen Target Circle binden kann und so als Primer in einer nachfolgenden Signalamplifikation mittels Rolling Circle Amplification fungieren kann;

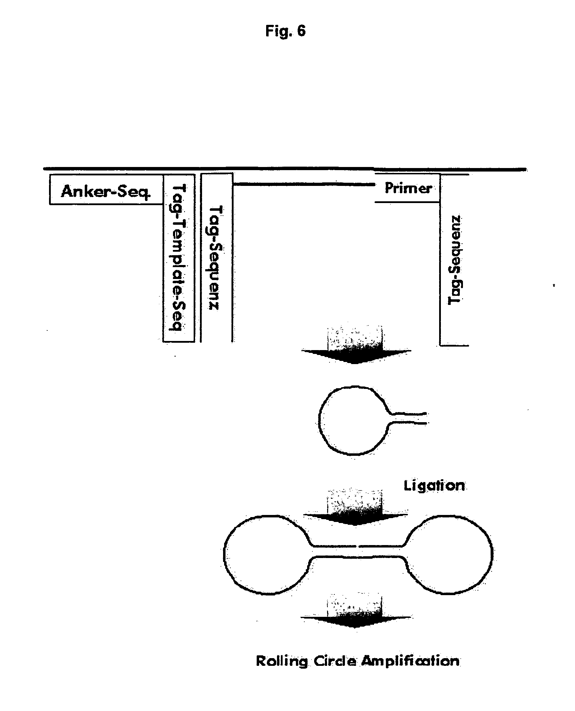

- Fig. 6: eine schematische Darstellung eines generisch anwendbaren, erfindungsgemäßen Verfahrens im Rahmen einerAmplifikation von DNA, bei der während der Synthese des neuen Nukleinsäurestranges ausgehend von RNA oder DNA mittels der Tag-Template-Sequenz des Anker-Oligonukleotids eine Tag-Sequenz am 3'-Ende der neu synthetisierten Nukleinsäure eingeführt wird, die komplementär zur Tag-Sequenz des Primers ist, sodass sich nach Denaturierung des neu synthetisierten Nukleinsäurestrangs vom Template-Strang intramolekulare Haamadelschlaufen-Strukturen bilden. Diese können für Ligationsreaktionen genutzt werden, um Nukleinsäuren in einer nachfolgenden Rolling Circle Amplification-Reaktion zu amplifizieren;
- Fg.7: die Versuchsergebnisse aus Beispiel 1;

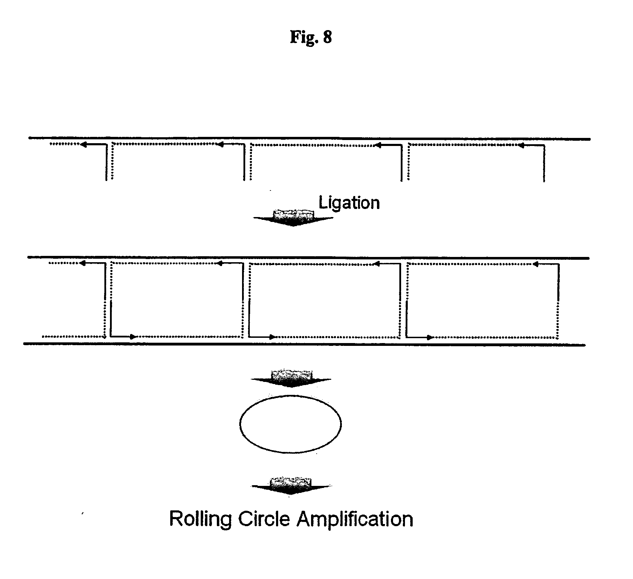

- Fig. 8: eine schematische Darstellung eines weiteren generisch anwendbaren, erfindungsgemäßen Verfahrens im Rahmen einer Amplifikation von DNA, bei der während der Synthese des neuen Nukleinsäurestranges ausgehend von RNA oder DNA mittels der Tag-Template-Sequenz des Anker-Oligonukleotids eine Tag-Sequenz am 3'-Ende der neu synthetisierten Nukleinsäure eingeführt wird. Weiterhin wird ein Primer verwendet, der eine Tag-Sequenz aufweist, die unterschiedlich zur oder gleich der Tag-Template-Sequenz des Anker-Oligonukleotids sein kann. Die entstehenden doppelsträngigen Enden werden ligiert, anschließend werden die neu synthetisierten Nukleinsäurestränge von den Template-Strängen getrennt, es bilden sich zirkuläre einzelsträngige Nukleinsäuren, die nachfolgend über Rolling Circle Amplification amplifiziert werden;

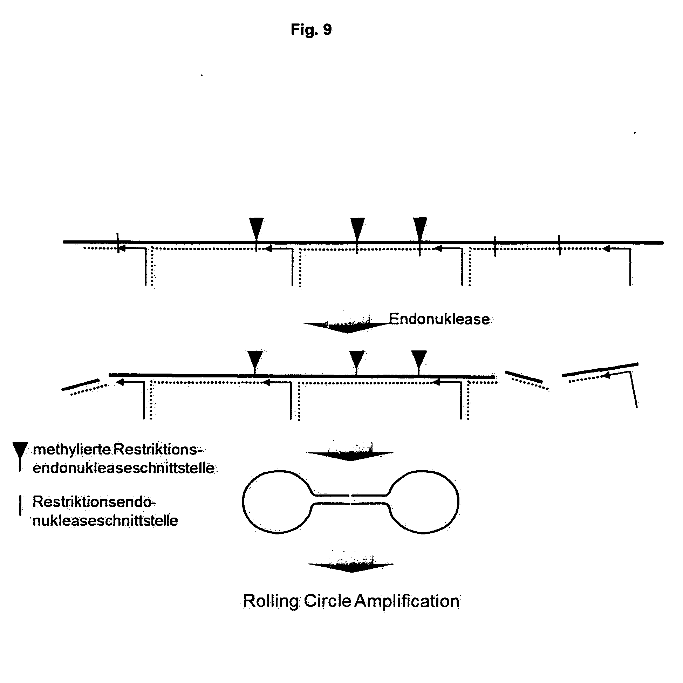

- Fig. 9: eine schematische Darstellung eines generisch anwendbaren, erfindungsgemäßen Verfahrens im Rahmen einerselektiven Amplifikation von methylierte DNA-Abschnitten (methylierte Basen sind durch Dreiecke gekennzeichnet). Während einer Nukleinsäure-Synthesereaktion ausgehend von DNA wird mittels der Tag-Template-Sequenz des Anker-Oligönukleotids eine Tag-Sequenz am 3'-Ende der neu synthetisierten Nukleinsäure eingeführt, die komplementär zur Tag-Sequenz des Primers ist. Nach der Nukleinsäure-Synthesereaktion wird die DNA mittels methylierungssensitiver Restriktionsendonukleasen geschnitten. Hiernach wird der neu synthetisierte Nukleinsäurestrang vom Template-Strang getrennt. In den nicht geschnitten Abschnitten der DNA, also den methylierten Bereichen, können sich nun intramolekulare Haamadelstrukturen ausbilden. Diese können für Ligationsreaktionen genutzt werden, um die Nukleinsäuren in einer nachfolgenden Rolling Circle Amplification-Reaktion zu amplifizieren;

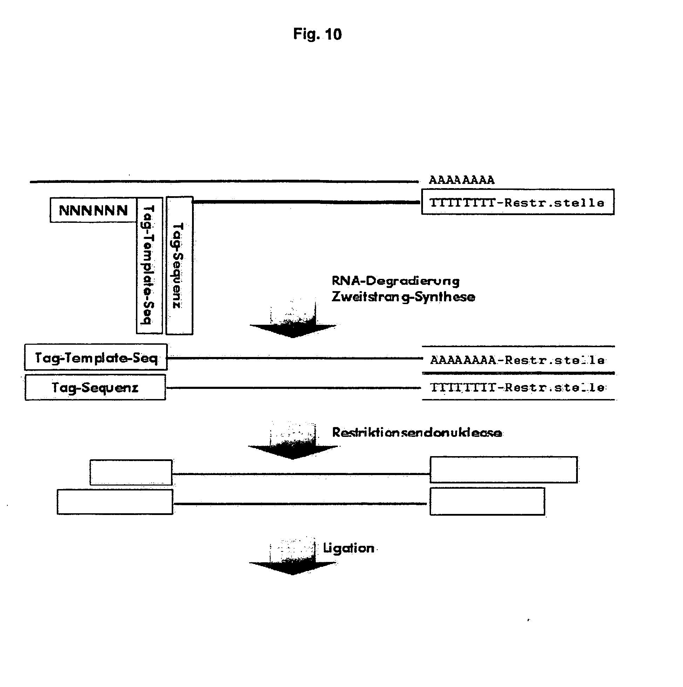

- Fig. 10: eine schematische Darstellung eines erfindungsgemäßen Verfahrens im Rahmen einerDNA-Klonierung, bei der während der Synthese des neuen Nukleinsäurestranges mittels der Tag-Template-Sequenz des Anker-Oligonukleotids die Schnittstelle für eine Restriktionsendonuklease eingeführt wird. Es folgt die Denaturierung von neu synthetisiertem Nukleinsäurestrang und Template-Strang, anschließend wird eine Zweitstrang-Synthese durchgeführt. Durch die Zweitstrangsynthese entsteht eine durch eine Restriktionsendonuklease erkennbare Doppelstrang-Sequenz;

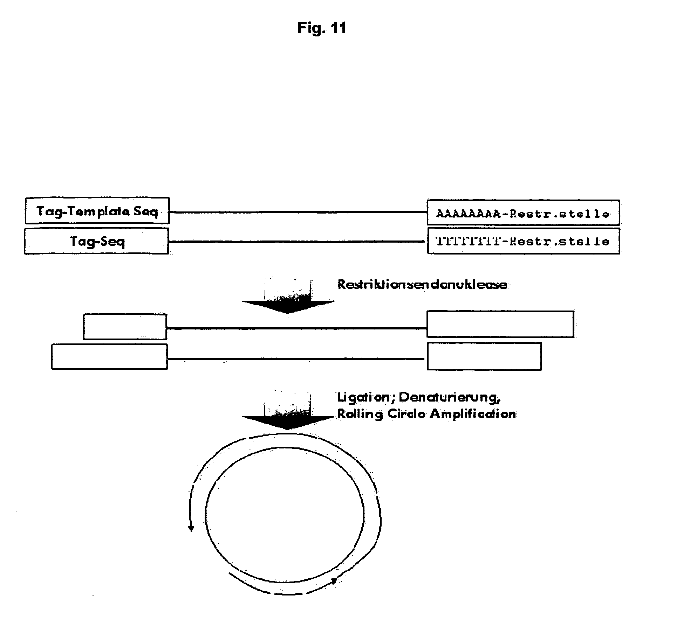

- Fig. 11: eine schematische Darstellung eines erfindungsgemäßen Verfahrens im Rahmen einerDNA-Amplifikation, bei der während der Synthese des neuen Nukleinsäurestranges mittels der Tag-Template-Sequenz des Anker-Oligonukleotids die Schnittstelle für eine Restriktionsendonuklease eingeführt wird. Der Primer enthält ebenfalls eine Restriktionsendonuklease-Schnittstelle. Durch Anker-Oligonukleotid und Primer werden kompatible Schnittstellen eingebracht. Es folgt die Denaturierung von neu synthetisiertem Nukleihsäurestrang und Template-Strang, anschließend wird eine Zweitstrang-Synthese durchgeführt. Durch die Zweitstrangsynthese entsteht eine durch eine Restriktionsendonuklease erkennbare Doppelstrang-Sequenz. Nach dem Restriktionsverdau beider Enden, nachfolgender intramolekularer Hybridisierung und anschließender Ligation entstehen doppelsträngige zirkuläre Nukleinsäuren, die z.B. nach Denaturierung mittels Rolling Circle Amplification amplifiziert werden können;

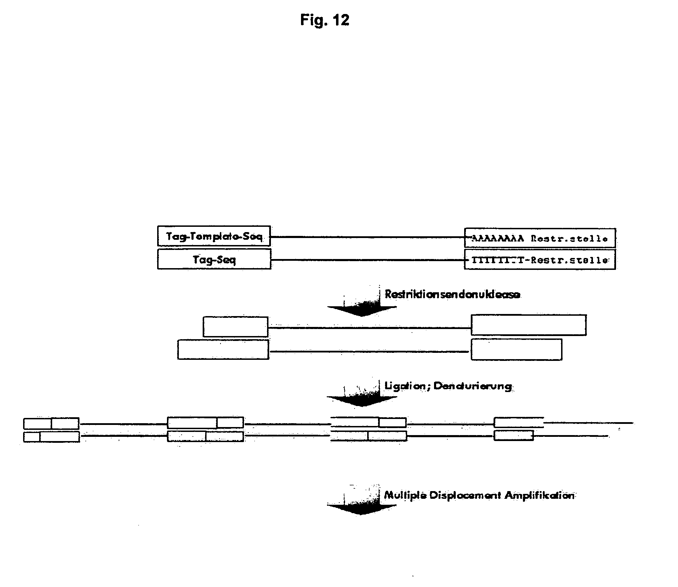

- Fig. 12: eine schematische Darstellung eines erfindungsgemäßen Verfahrens im Rahmen einerDNA-Amplifikation, bei der während der Synthese des neuen Nukleinsäurestranges mittels der Tag-Template-Sequenz des Anker-Oligonukleotids die Schnittstelle für eine Restriktionsendonuklease eingeführt wird. Der Primer enthält ebenfalls eine Restriktionsendonuklease-Schnittstelle. Durch Anker-Oligonukleotid und Primer werden kompatible Schnittstellen eingebracht Es folgt die Denaturierung von neu synthetisiertem Nukleinsäurestrang und Template-Strang, anschließend wird eine Zweitstrang-Synthese durchgeführt. Durch die Zweitstrangsynthese entsteht eine durch eine Restriktionsendonuklease erkennbare Doppelstrang-Sequenz Nach dem Restriktionsverdau beider Enden, nachfolgender intramolekularer Hybndisierung und anschließender Ligation entstehen doppelsträngige lineare Nukleinsäuren, die z.B. nach Denaturierung mittels Multiple Displacement Amplification (MDA) amplifiziert werden können;

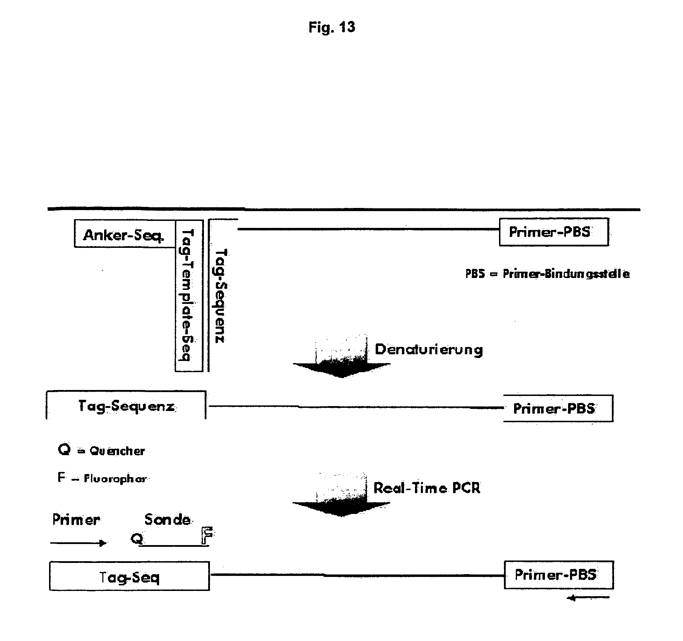

- Fig. 13: eine schematische Darstellung eines erfindungsgemäßen Verfahrens im Rahmen einerNukleinsäure-Detektionsreaktion mittels Reat-Time PCR, bei der während der Synthese des neuen Nukleinsäurestranges mittels der Tag-Template-Sequenz des Anker-Oligonukleotids eine Sonden- und eine Primer-Bindestelle eingeführt wird,

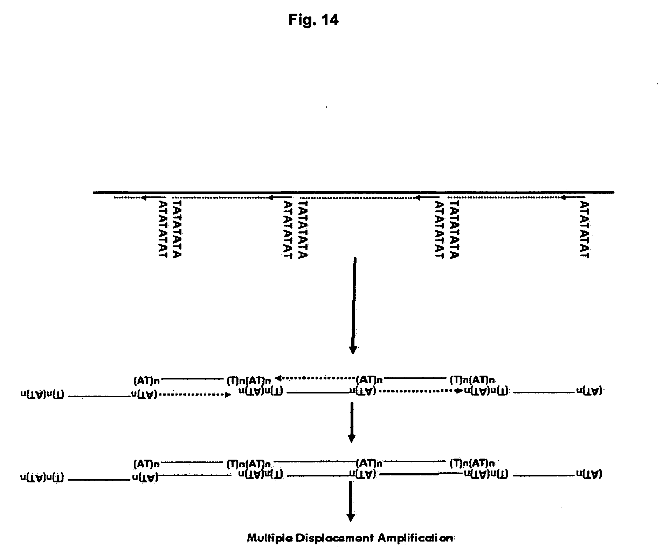

- Fig. 14: eine schematische Darstellung eines erfindungsgemäßen Verfahrens im Rahmen einerFusion von Nukleinsäure-Molekülen, bei der während der Synthese des neuen Nukleinsäuresttanges mittels der Tag-Template-Sequenz des Anker-Oligonukleotids eine Sequenz in den neu synthetisierten Strang eingeführt wird, die mit sich selbst komplementär ist, das heißt, dass das 3'-Ende des neu synthetisierten Nukleinsäurestranges mit dem 3' eines weiteren neu synthetisierten Nukleinsäurestranges hybridisieren kann. Die Tag-Sequenz des Primers ist in gleicher Weise gestaltet, sodass das 5'-Ende des neu synthetisierten Nukleinsäurestranges mit dem 5' eines weiteren neu synthetisieren Nukleinsäurestranges hybndisieren kann. Im vorliegenden Fall sind Tag-Template-Sequenz des Anker-Oligonukleotids und Tag-Sequenz des Primers identisch ( (AT)ₙ ). Es können sich lineare oder zirkuläre Hybridisierungsstrukturen bilden. Die einzelsträngigen Bereiche werden nachfolgend mittels einer Polymerase aufgefüllt, die Einzelstrang-Nicks werden ligiert Eine anschließende Multiple Displacement Amplification- oder Rolling Circle Amplification-Reaktion kann zur Vervielfältigung der Sequenzen genutzt werden;

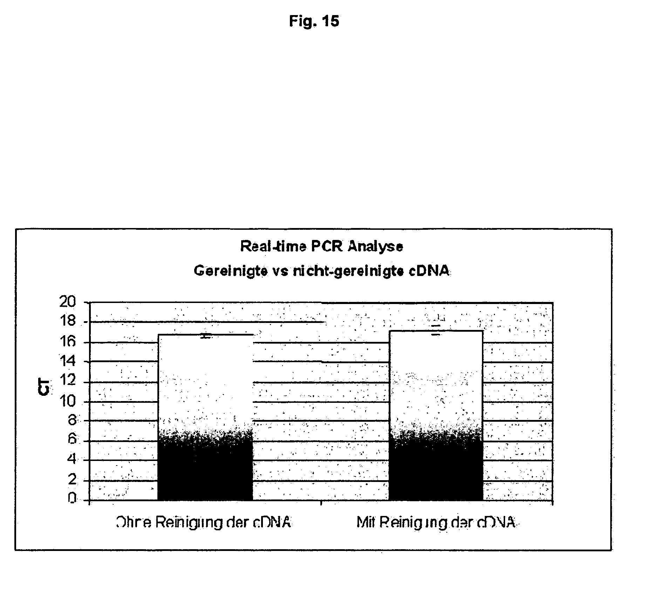

- Fig. 15: ein Balkendiagramm der Real-Time PCR-Analyse aus Beispiel 2;

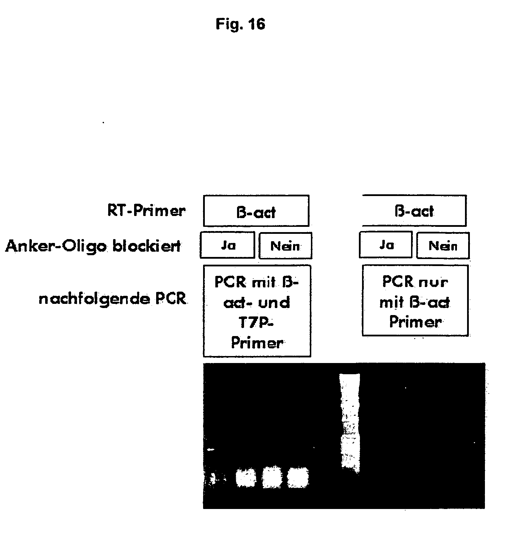

- Fig. 16: eine Gel-Analyse der Amplifikate aus Beispiel 3;

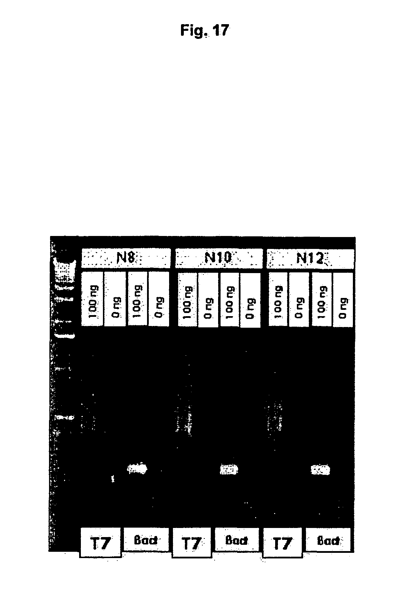

- Fig. 17: eine Gel-Analyse der Amplifikate aus Beispiel 4;

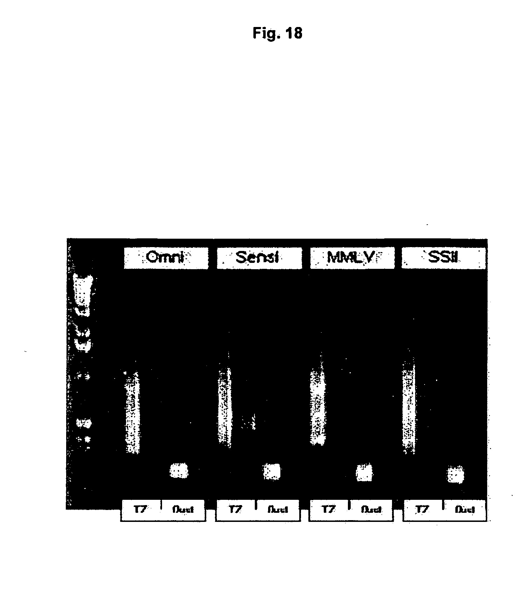

- Fig. 18: eine Gel-Analyse aus Beispiel 5;

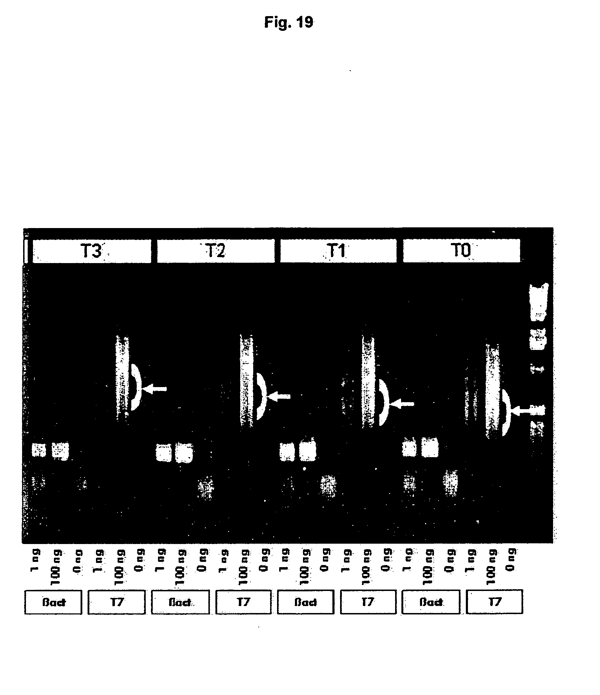

- Fig. 19: eine Gel-Analyse aus Beispiel 6;

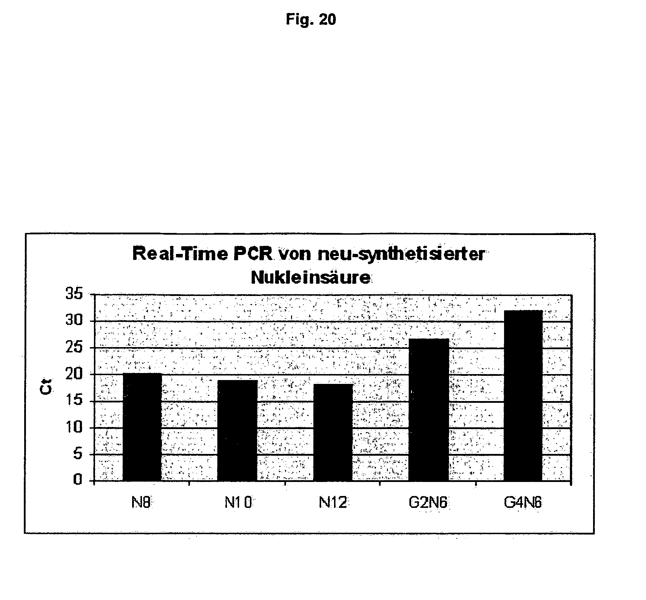

- Fig. 20: ein Balkendiagramm mit dem Ergebnis einer Real-Time-Amplifikation aus Beispiel 7;

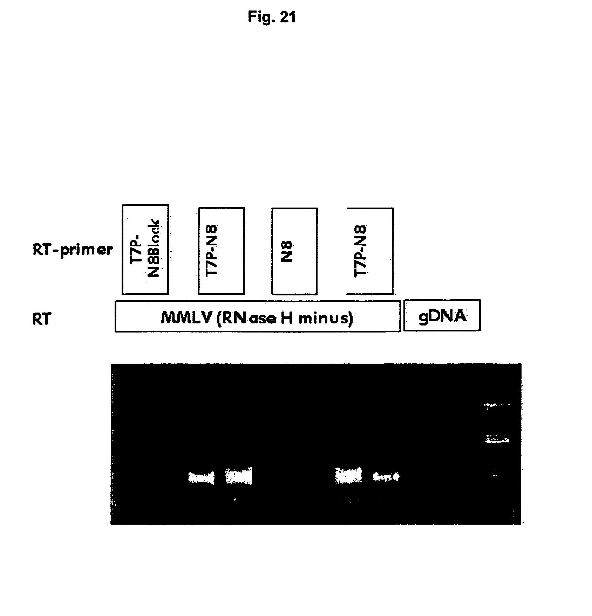

- Fig. 21: eine Gel-Analyse aus Beispiel 8; und

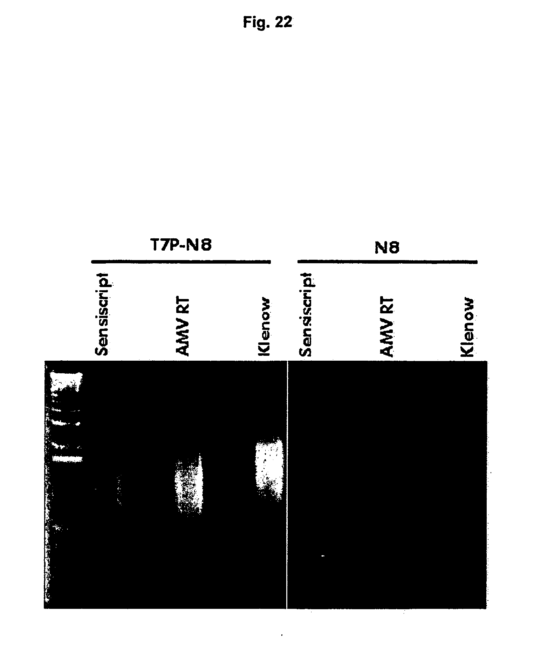

- Fig. 22: eine Gel-Analyse aus Beispiel 9.

Fig. 1 zeigt schematisch den Einbau einer Tag-Sequenz während der Synthese des neuen Nukleinsäurestrangs durch eine DNA-Polymerase. Das Anker-Oligonukleotid weist eine Anker-Sequenz auf (durch "NNNNNN" gekennzeichnet). Die Basen der Anker-Sequenz hybridisieren mit einem entsprechend komplementären Abschnitt der Template-Nukleinsäure. Der Primer dient zum Starten der Synthese des neuen Nukleinsäurestrangs. Stößt die DNA-Polymerase im Verlauf der Synthese auf das Anker-Oligonukleotid, so wird die Synthese des neuen Nukleinsäurestrangs an der Tag-Template-Sequenz des Anker-Oligonukleotids durch Template-Switch fortgeführt, wodurch die zur Tag-Template-Sequenz komplementäre Tag-Sequenz in den neu synthetisierten Nukleinsäurestrang eingebaut wird.

Zur weiteren Klarstellung der vorliegenden Erfindung werden im Folgenden einige Ausführungsformen beispielhaft dargestellt.

### Ausführungsform 1 (nicht zur Erfindung gehörend)

Diese Ausführungsform betrifft eine erfindungsgemäße Amplifikation von RNA aus geringen Ausgangsmengen. Eine schematische Darstellung dieser Verfahrensvariante Ist in Fig. 2 zu finden.

Es werden 100 ng einer total-RNA mit Omniscript^{®} RT Kit (QIAGEN GmbH, Hilden, Deutschland) revers transkribiert. Die Reverse Transkriptase-Reaktion (RT-Reaktion) wird in Gegenwart von Nukleotiden, 1 µM oligo-dT Primer und 10 µM Anker-Oligonukleotid durchgeführt. Die Tag-Template-Sequenz des Anker-Oligonukleotids enthält die Sequenz für einen RNA-Polymerase-Promotor (hier T7-RNA-Polymerase-Promotor) im 5'-Bereich und eine 8 Basen lange Zufallssequenz (random sequence) im Bereich des 3'-Endes. Anschließend wird die cDNA in einer *in vitro* Transkription nach Protokoll des MegaScript Kits^{®} (Ambion (Europe) Ltd., Huntington, UK) eingesetzt. Hierbei wird die geringe Ausgangsmenge an total-RNA in sense Richtung stark amplifiziert.

In Abwandlung zur oben beschriebenen Versuchsdurchführung kann z.B. ein Anker-Oligonukleotid auch derart verwendet werden, dass das Anker-Oligonukleotid neben dem Einbau einer Tag-Sequenz auch den Start der DNA-Synthese vermittelt, also der mit der Template-Nukleinsäure hybridisierende Teil (Anker-Sequenz) als Primer für den neu synthetisierten Strang dient. In diesem Fall muss das Anker-Oligonukleotid ein 3'-Ende, wie z.B. ein freies 3'-OH-Ende, enthalten, das durch die Polymerase verlängerbar ist.

In einer weiteren Abwandlung zur beschriebenen Versuchsdurchführung kann z.B. ein Anker-Oligonukleotid eine spezifische oder degenerierte Sequenz im Bereich der Anker-Sequenz enthalten, so dass nur eine Sequenz oder eine bestimmte Gruppe von Sequenzen amplifiziert werden kann. In einer weiteren Abwandlung können anstatt des Oligo-dT Primers auch Random-Primer, degenerierte Primer oder spezifische Primer verwendet werden.

In einer weiteren Abwandlung zur oben beschriebenen Versuchsdurchführung kann anstelle der RNA auch genomische DNA oder eine andere DNA als Ziel-Nukleinsäure eingesetzt werden. Dadurch kann z.B. jede beliebige Sequenz des Genoms in RNA umgewandelt werden.

### Ausführungsform 2 (nicht zur Erfindung gehörend)

Auch diese Ausführungsform befasst sich mit einer erfindungsgemäßen Amplifikation von RNA. Im Gegensatz zur RNA-Amplifikation gemäß Ausführungsform 1 wird hier neben der Tag-Sequenz am 3'-Ende der neu synthetisierten Nukleinsäure, die mittels Tag-Template-Sequenz des Anker-Oligonukleotids eingeführt wurde, weiterhin eine Tag-Sequenz am 5'-Ende der neu synthetisierten Nukleinsäure mittels eines Primers mit Tag-Sequenz an seinem 5'-Ende eingeführt. Die Tag-Sequenz des Primers sowie die Tag-Template-Sequenz des Anker-Oligonukleotids enthalten beide die Sequenz des Promotors. Dieses führt dazu, dass die Tag-Sequenzen am 3'-Ende und am 5'-Ende der neu synthetisierten Nukleinsäure im Bereich des Promotors komplementär zueinander sind. Nach Hybridisierung des 3'-Endes der neu synthetisierten Nukleinsäure mit dem komplementären 5'-Ende des neu synthetisierten Nukleinsäurestrangs kann an den nun doppelsträngigen Promotor die RNA-Polymerase binden und die *in vitro* Transkription wird Initiiert. Eine schematische Darstellung einer RNA-Amplifikation, bei der während der Erststrang-cDNA Synthese die Promotor-Sequenz mittels eines Anker-Oligonukleotids in den neu synthetisierten Nukleinsäurestrang eingeführt wird, ist in Fig. 3 beschrieben.

100 ng einer total-RNA werden mit Omniscript^{®} (QIAGEN) revers transkribiert. Die Reverse Transkriptase Reaktion (RT-Reaktion) wird in Gegenwart von RT-Puffer, Nukleotiden (0,5 mM) und Anker-Oligonukleotiden (10 µM) durchgeführt Die Tag-Template-Sequenz des Anker-Oligonukleotids enthält die Sequenz für den T7-RNA-Polymerase-Promotor im 5'-Bereich und eine 8 Basen lange Zufallssequenz im 3'-Bereich. In dieser Reaktion fungiert das Anker-Oligonukleotid gleichzeitig als Primer, d.h., dass die Tag-Template-Sequenz des Anker-Oligonukleotids in dem Fall, dass das Anker-Oligonukleotid selbst als Primer dient, als Tag-Sequenz des Primers zu betrachten ist. Nach der RT-Reaktion von 60 min Dauer bei 37°C wird das Reaktionsgemisch für 5 min auf 95°C erhitzt. Anschließend wird die cDNA in einer *in vitro* Transkription nach Protokoll des MegaScript^{®} Kits (Ambion) eingesetzt. Hierbei wird die geringe Ausgangsmenge an total-RNA in sense Richtung stark amplifiziert.

In Abwandlung zur oben beschriebenen Versuchsdurchführung kann z.B. auch ein Anker-Oligonukleotid mit alternativen RNA-Polymerase-Promotoren verwendet werden.

In einer zweiten Abwandlung zur oben beschriebenen Versuchsdurchführung kann z.B. auch ein Anker-Oligonukleotid eine spezifische oder degenerierte Sequenz im Bereich der Anker-Sequenz haben, so dass nur ein Transkript oder eine bestimmte Gruppe von Transkripten amplifiziert werden kann.

In einer dritten Abwandlung zur oben beschriebenen Versuchsdurchführung kann anstelle der RNA auch genomische DNA oder eine andere DNA als Ziel-Nukleinsäure eingesetzt werden. Dadurch kann z.B. jede beliebige Sequenz des Genoms in RNA umgewandelt werden.

### Ausführungsform 3 (nicht zur Erfindung gehörend)

Ausführungsform 3 betrifft wiederum eine RNA-Amplifikation, die eine Abwandlung der in Ausführungsform 2 beschriebenen RNA-Amplifikation darstellt. Der Unterschied besteht darin, dass der neu synthetisierte Nukleinsäurestrang zu einer Haamadeistruktur hybridisiert mit einem 3'-Überhang. Dieser wird mittels der Tag-Template-Sequenz des Anker-Oligonukleotids in die neu synthetisierte Nukleinsäure eingeführt. Durch die Verwendung eines 3'-Überhangs führt die RNA-Polymerase bei der Transkription am 5' rezessiven Ende einen so genannten Template-switch durch, was zu einem RNA-Molekül führt, das mehrere Kopien derselben Sequenz hintereinander enthält (Konkatemer). In Fig. 4 ist eine schematische Darstellung einer RNA-Amplifikation gezeigt, bei der während der Erststrang-cDNA-Synthese die Promotorsequenz mittels der Tag-Template-Sequenz des Anker-Oligonukleotids eingeführt wird. Hierbei weist die Tag-Template-Sequenz 5' der Promotor-Sequenz mindestens eine weitere Base auf, die bei der Hybridisierung des 3'-Endes und des 5'-Endes der neu synthetisierten Nukleinsäure über das 5'-Ende hinausragt.

100 ng einer total-RNA werden mit Omniscript^{®} (QIAGEN) revers transkribiert. Die Reverse Transkriptase Reaktion (RT-Reaktion) wird in Gegenwart von RT-Puffer, Nukleotiden (0,5 mM), einem Anker-Oligonukleotid (10 µM) und einem Primer durchgeführt. Die Tag-Sequenz des Primers und die Tag-Template-Sequenz des Anker-Oligonukleotids sind identisch, sie enthalten den T7-RNA-Polymerase-Promotor, jedoch weist die Tag-Template-Sequenz des Anker-Oligonukleotids am 3'-Ende 3 zusätzliche Nukleotide auf. Nach der 60 min langen RT-Reaktion bei 37°C wird das Reaktionsgemisch für 5 min auf 95°C erhitzt. Anschließend wird die cDNA in einer *in vitro* Transkription nach Protokoll des MegaScript^{®} Kits (Ambion) eingesetzt. Hierbei wird die geringe Ausgangsmenge an total-RNA in sense Richtung stark amplfiziert.

In Abwandlung zur oben beschriebenen Versuchsdurchführung kann z.B. ein Anker-Oligonukleotid mit alternativen RNA-Polymerase-Promotoren verwendet werden.

Gemäß einer zweiten Abwandlung zur beschriebenen Versuchsdurchführung kann z.B. ein Anker-Oligonukleotid eine spezifische oder degenerierte Sequenz im Bereich der Anker-Sequenz aufweisen, so dass nur ein Transkript oder eine bestimmte Gruppe von Transkripten amplifiziert werden kann.

In einer dritten Abwandlung zur beschriebenen Versuchsdurchführung kann anstelle der RNA auch genomische DNA oder eine andere DNA als Ziel-Nukleinsäure eingesetzt werden. Dadurch kann z.B. jede beliebige Sequenz des Genoms in RNA umgewandelt werden.

### Ausführungsform 4 (nicht zur Erfindung gehörend)

Diese Ausführungsform befasst sich mit einem Anker-Oligonukleotid für die Signal-Amplifikation mittels einer so genannten Rolling Circle Amplification (RCA). Ziel bei diesem Verfahren ist es, ein Signal ausgehend von einer bestimmten Sequenz oder einer Schar von Sequenzen durch eine *in vitro* DNA-Synthese zu verstärken. Es kann DNA oder RNA als Template-Nukleinsäure verwendet werden. Im efindungsgemäßen Verfahren wird ein Anker-Oligonukleotid benötigt, das eine Anker-Sequenz aufweist, die an der Template-Nukleinsäure hybridisieren kann, wobei die Tag-Template-Sequenz des Anker-Oligonukleotids identisch zu einem Sequenzabschnitt eines zirkulären Nukleinsäuremoleküls (Target Circle) ist, sodass die mittels Tag-Template-Sequenz des Anker-Oligonukleotids in den neu synthetisierten Strang eingeführte Tag-Sequenz mit seinem 3'-Ende mit dem Target Circle hybridisiert und dort in einer nachfolgenden RCA als Primer dient (schematische Darstellung in Fig. 5).

Es werden 100 ng total-RNA in einer RT-Reaktion mit Omniscript^{®} (QIAGEN), RT-Puffer, dNTPs, 1 µM ß-act-Primer (in Fig. 5 mit "Spec.Seq." bezeichnet) und einem ß-act-Anker-Oligonukleotid (in Fig. 5 ist die ß-act Sequenz des Anker-Oligonukleotids mit "Anker" bezeichnet) nach dem Omniscript^{®} Standard-Protokoll in cDNA umgeschrieben. Das ß-act-Anker-Oligonukleotid weist einerseits eine ß-Aktin-Transkript-bindende Anker-Sequenz und andererseits eine Tag-Template-Sequenz auf, die aus einzelsträngiger M13-Phagen-DNA stammt. Die Sequenz des ß-Aktin-Primers bindet 3' in Bezug auf die Bindestelle des ß-act Anker-Oligonukleotids im Transkript. Bei der cDNA-Synthese wird die Tag-Sequenz eingebaut, die als Primer bei der Rolling-Circle-Amplifikation der M13-Phagen-DNA dient. Die cDNA wird mittels QIAquick^{®} (QIAGEN) nach Standard-Protokoll gereinigt. Die Rolling-Circle-Amplifikation wird in einem 50 µl Reaktionsansatz durchgeführt, der Phi29-DNA-Polymerase, Puffer, Nukleotide und M13-DNA enthält. Hierbei wird die. Ausgangsmenge an M13-DNA stark amplifiziert.

In Abwandlung zur oben beschriebenen Versuchsdurchführung kann z.B. ein Anker-Oligonukleotid derart verwendet werden, dass die Tag-Sequenz am 3'-Ende der neu synthetisierten Nukleinsäure alternative Target Circles bindet und dort bei diesen als Primer dient.

In einer zweiten Abwandlung der oben beschriebenen Versuchsdurchführung können Anker-Oligonukleotide mit Random, degenerierten oder anderen spezifischen Sequenzen als Anker-Sequenz verwendet und/oder die Synthese des neuen Nukleinsäurestrangs von Random, degenerierten, anderen spezifischen oder OligodT Primern gestartet werden.

Gemäß einer dritten Abwandlung der oben beschriebenen Versuchsdurchführung kann anstelle der RNA als Template-Nukleinsäure auch genomische DNA oder eine andere DNA eingesetzt werden.

### Ausführungsform 5

Diese weitere Ausführungsform betrifft eine DNA-Amplifikation. Diese Ausführungsform der gegenständlichen Erfindung entspricht zunächst den in Beispiel 1 (siehe unten) dargestellten Schritten. Wederum wird hier neben der Tag-Sequenz am 3'-Ende der neu synthetisierten Nukleinsäure, die mittels Tag-Template-Sequenz des Anker-Oligonukleotids, eingeführt wurde, weiterhin eine Tag-Sequenz am 5'-Ende der neu synthetisierten Nukleinsäure mittels eines Primers mit Tag-Sequenz an seinem 5'-wende eingeführt. In dem vorliegenden Ausführungsform findet jedoch im Gegensatz zu Beispiel 1 vor der Ligationsreaktion keine Trennung des neu synthetisierten Nukleinsäurestrangs von der Template-Nukleinsäure statt. Die Ligationsprodukte bestehen wie auch in Beispiel 1 aus zwei Sequenzen, die an den Tag-Sequenzen fusioniert sind. Diese können nach einer Strang-Trennung über eine RCA arnplifiziertwerden (schematische Darstellung in Fig. 8).

100 ng einer gDNA wird denaturiert. An den Einzelsträngen wird eine Polymerase Reaktion, hier mit Omniscript® (QIAGEN), durchgeführt. Die Polymerase Reaktion wird in Gegenwart eines geeigneten Puffers, Nukleotiden (0,5 mM), Primern und Anker-Oligonukleotiden (10 µM) durchgeführt. Die Tag-Template-Sequenz des Anker-Oligonukleotids enthält eine spezifische Sequenz (hier einen T7-Promotor) im 5'-Bereich und eine 8 Basen lange Züfallssequenz im 3'-Bereich. Der eingesetzte Primer und das Anker-Oligonukleotid weisen in diesem Ausführungsform eines identische Sequenz auf. Nach der Reaktion von 60 min bei 37°C wird die DNA in einer Ligation (T4-Ligase) ligiert. Die entstehenden Ligationsprodukte werden auf 95°C erhitzt und anschließend in einer RCA-Reaktion mit dem REPLI-g^{®} Kit (QIAGEN) amplifiziert. Hierbei wird die Ausgangsmenge an gDNA stark amplifiziert.

In Abwandlung zur oben beschriebenen Versuchsdurchführung kann z.B. ein Anker-Oligonukleotid mit alternativen Tag-Template-Sequenzen verwendet werden.

In einer zweiten Abwandlung zur beschriebenen Versuchsdurchführung kann z.B. ein Anker-Oligonukleotid eine spezifische oder degenerierte Sequenz im Bereich der Anker-Sequenz aufweisen, so dass nur eine Sequenz oder eine bestimmte Gruppe von Sequenzen amplifiziert werden kann. Die Anker-Sequenz kann auch eine alternative Länge aufweisen.

Auch das unten beschriebene Beispiel 1 kann weitere Ausführungsformen aufweisen. In Abwandlung zu der in Beispiel 1 beschriebenen Versuchsdurchführung kann z.B. ein Anker-Oligonukleotid mit alternativen Tag-Template-Sequenzen verwendet werden.

In einer weiteren Abwandlung der in Beispiel 1 beschriebenen Versuchsdurchführung kann z.B. ein Anker-Oligonukleotid eine spezifische oder degenerierte Sequenz im Bereich der Anker-Sequenz aufweisen, sodass nur eine Sequenz oder eine bestimmte Gruppe von Sequenzen amplifiziert werden kann. Die Anker-Sequenz kann auch eine alternative Länge aufweisen.

In einer dritten Abwandlung der in Beispiel 1 beschriebenen Versuchsdurchführung kann während der Ligase-Reaktion eine DNA-Polymerase zugegen sein. Diese kann neben der DNA-Polymerase-Aktivität auch noch eine Exonukleaseaktivität enthalten. Im Falle, dass sich, beispielsweise bei großer Länge der neu synthetisierten Nukleinsäure, keine blunt ends sondern sticky ends bei der intramolekularen Hybridisierung bilden, können die entstehenden Lücken durch die Polymerase aufgefüllt werden.

In einer vierten Abwandlung der in Beispiel 1 beschriebenen Versuchsdurchführung kann während der Ligase-Reaktion ein Oligonukleotid zugegen sein, dass durch Intramolekulare Hybridisierung eine blunt end Haamadelstruktur ausbildet und anschließend mit dem blunt end der neu synthetisieren und zur Haamadelstruktur hybridisierten Nukleinsäure zum geschlossenen zirkulären Nukleinsäuremolekül ligiert wird.

### Ausführungsform 6 (nicht zur Erfindung gehörend)

Diese Ausführungsform betrifft die DNA-Amplifikation von methylierten DNA-Abschnitten. Diese Form der Amplifikation beginnt entsprechend Beispiel 1 (siehe unten). In der vorliegenden Ausführungsform wird hier neben der Tag-Sequenz am 3'-Ende der neu synthetisierten Nukleinsäure, die mittels Tag-Template-Sequenz des Anker-Oligonukleotids eingeführt wurde, weiterhin eine Tag-Sequenz am 5'-Ende der neu synthetisierten Nukleinsäure mittels eines Primers mit Tag-Sequenz an seinem 5'-Ende eingeführt. Die Tag-Sequenz des Primers sowie die Tag-Template-Sequenz des Anker-Oligonukleotids enthalten eine identische Sequenz. Dieses führt dazu, dass die Tag-Sequenzen am 3'-Ende und am 5'-Ende der neu synthetisierten Nukleinsäure komplementär zueinander sind. Nachfolgend der Synthese des neuen Nukleinsäürestrangs, der komplementär zur partiell methylierten DNA ist, wird mit methylierungssensitiven Restriktionsendonukleasen geschnitten. Anschließend erfolgt die Strangtrennung durch eine Inkubation von 5 min bei 95°C.

Nach Hybridisierung des 3'-Endes der neu synthetisierten Nukleinsäure mit dem komplementären 5'-Ende des neu synthetisierten Nukleinsäurestrangs bildet sich bei nicht geschnittenen neu synthetisierten Nukleinsäuresträngen eine Haarnadetstruktur aus, die ein glattes doppelsträngiges Ende enthält. Zwei solcher Haarnadelschlaufen werden in einer "Blunt"end"-Ligation ligiert, so dass ein hantelförmiges, zirkuläres DNA-Molekül entsteht. Hierdurch wird methylierte. DNA mittels RCA amplifiziert während nicht methylierte DNA nicht amplifiziert werden kann (schematische Darstellung in Fig. 9).

In einer vierten Abwandlung der Versuchsdurchführung kann während der Ligase-Reaktion ein Oligonukleotid zugegen sein, dass durch intramolekulare Hybridisierung eine blunt end Haarnadelstruktur ausbildet und anschließend mit dem blunt end der neu synthetisierten und zur Haamadeistruktur hybridisierten Nukleinsäure zum geschlossenen zirkulären Nukleinsäuremolekül ligiert wird.

100 ng einer gDNA wird denaturiert. An den Einzelsträngen wird eine Polymerase-Reaktion mit Omniscript^{®} (QIAGEN) durchgeführt. Die Polymerase- Reaktion wird in Gegenwart eines geeigneten Puffers, Nukleotiden (0,5 mM), Primem und Anker-Oligonukleotiden (10 µM) durchgeführt, Die Tag-Template-Sequenz der Anker-Oligonukleotide enthält eine spezifische Sequenz (hier einen T7-Promotor) im 5'-Bereich und eine 8 Basen lange Zufallssequenz im 3'-Bereich. Die Primer weisen eine zu den Anker-Oligonukleotiden identische Sequenz auf. Nach einer Stunde bei 37°C wird die DNA mit einer methylierungssensitiven Restriktionsendonuklease behandelt, in der vorliegenden Ausführungsform mit Hpa II, so dass nur nichtmethylierte Bereiche verdaut werden können, methylierte Bereiche aber Intakt bleiben. Nachfolgend wird die DNA für 5 min auf 95°C erhitzt und so denaturiert und anschließend in einer Ligation (T4-Ligase) ligiert. Die entstehenden Ligationsprodukte werden in einer RCA-Reaktion mit dem REPLI-g^{®} Kit (QIAGEN) amplifiziert Hierbei wird die Ausgangsmenge an methylierter DNA stark amplifiziert .

In Abwandlung zur Verwendung des beschriebenen Restriktionsenzyms Hpa II können auch andere Enzyme verwendet werden, wie z.B. AatII, BcnI, Bsh1236I, Bsh1285I, BshTI, Bsp68I, Bsp119I, Bsp143II, Bsu15I, CseI, Cfr10I, Cfr42I, CpoI, Eco47III, Eco52I, Eco72I, Eco88I, Eco105I, EheI, Esp3I, FspAI, Hhal, Hin1I, Hin6I, Hpall, Kpn2I, MbiI, Mlul, Notl, NsbI, Paul, Pdil, Pfl23II, Psp1406I, PvuI, Sall, Sgsl, SmaI, Smul, SsiI, TaiI, Taul, XhoI, etc.

Gemäß einer weiteren Abwandlung der beschriebenen Versuchsdurchführung wird die DNA nicht mit einer methylierungssensitiven Restriktionsendonuklease verdaut, sondern mit einem Isoschizomer, das methylierte und nicht-methylierte DNA verdaut (z.B. Isoschizomer von Hpall ist MspI). Eine Amplifikation der Ligationsprodukte führt zur Ampliflkation der residuellen DNA.

### Ausführungsform 7 (nicht zur Erfindung gehörend)

Diese Ausführungsform betrifft die Verwendung der erfindungsgemäßen Anker-Oligonukleotide für eine DNA-Klonierung, bei der während der Synthese des neu synthetisierten Nukleinsäurestranges mittels der Tag-Template-Sequenz des Anker-Oligonukleotide die Schnittstelle für eine Restriktionsendonukfease eingeführt wird. Es folgt die Denaturierung von neu synthetisiertem Nukleinsäurestrang und Template-Strang, anschließend wird eine Zweitstrang-Synthese durchgeführt Durch die Zweitstrangsynthese entsteht eine durch eine Restriktionsendonuklease erkennbare Doppelstrang-Sequenz.

Es kann DNA oder RNA als Template-Nukleinsäure verwendet werden. Hierzu kann ausgehend von einem ersten Primer die DNA-Synthese bis hin zum - nach Template Switch - 5'-Ende des Anker-Oligonukleotids durchgeführt werden, wobei durch das Anker-Oligonukleotid eine Tag-Sequenz am 3'-Ende der neu synthetisierten Nukleinsäure eingeführt wird. Bei einigen Ausführungsformen bedarf es einer Restriktionsschnittstelle in der Sequenz dieses ersten Primers. Das Anker-Oligonukleotid enthält bei dieser Ausführungsform zusätzlich in seiner Sequenz eine oder mehrere Restriktionsschnittstellen, wodurch eine Ligation nach Restriktionsverdau ermöglicht werden kann. Beispielsweise kann eine Ligation mit einer geschnittenen Vektor-DNA z.B. zu Klonierungszwecken erfolgen.

1 µg total-RNA wird in einer RT-Reaktion mit Reverser Transkriptase, RT-Puffer, dNTPs, 1 µM Oligo-dT-Primer (enthaltend die Sequenz einer NotI-Schnittstelle am 5'-Ende) und 10 µM eines Anker-Oligonukleotids (enthaltend die Sequenz einer Notl-SchnitfStelle am 5'-Ende) nach dem Omniscript^{®} Standard-Protokoll in cDNA umgeschrieben. Das Anker-Oligonukleotid hat zum einen eine Zufallssequenz als hybridisierenden Anker. Bei der cDNA-Synthese wird eine Tag-Sequenz mittels des Anker-Oligonukleotids am 3'-Ende des neu synthetisierten Nukleinsäurestrangs eingebaut, welche die Notl-Schnittstelle enthält. Nachfolgend wird der Zweitstrang der cDNA über eine Standard-Reaktion mit Klenow-Fragment, dNTPs und einem Primer, dessen Sequenz identisch zu einem Teil der Tag-Template-Sequenz des eingesetzten Anker-Oligonukleotids ist, erzeugt. Die cDNA wird über QIAquick^{®} (QIAGEN) nach Standard-Protokoll gereinigt und anschließend mit NotI geschnitten. Anschließend erfolgt die Ligation mit einem mit NotI geschnittenen Vektor (schematische Darstellung in Fig. 10). Der Vektor wird anschließend in kompetente E.coli Zellen transformiert. Die Übernachtkultur wird nach bekannten Verfahren aufgereinigt und lysiert und das Plasmid anschließend isoliert Auf diese Weise wird die Ausgangsmenge an der aus der eingesetzten RNA gewonnenen cDNA amplifiziert.

In Abwandlung zur oben beschriebenen Versuchsdurchführung kann z.B. ein Anker-Oligonukleotid mit spezifischer oder degenerierter Anker-Sequenz verwendet werden.

Gemäß einer weiteren Abwandlung der oben beschriebenen Versuchsdurchführung kann die cDNA-Synthese von Random-Primem oder spezifischen Primem gestartet werden, die an Ihrem 5'-Ende eine Schnittstelle für ein Restriktionsenzym tragen.

### Ausführungsform 8 (nicht zur Erfindung gehörend)

Diese Ausführungsform betrifft die Verwendung der erfindungsgemäßen Anker-Oligonukleotide für eine DNA-Amplikation, bei der während der Synthese des neu synthetisierten Nukleinsäurestranges mittels der Tag-Template-Sequenz des Anker-Oligonukleotids die Schnittstelle für eine Restriktionsendonuklease eingeführt wird. Es folgt die Denaturierung von neu synthetisiertem Nukleinsäurestrang und Template-Strang, anschließend wird eine Zweitstrang-Synthese durchgeführt. Durch die Zweitstrangsynthese entsteht eine durch eine Restriktionsendonuklease erkennbare Doppelstrang-Sequenz.

Es kann DNA oder RNA als Template-Nukleinsäure verwendet werden. Hierzu kann ausgehend von einem ersten Primer die DNA-Synthese bis hin zum - nach Template Switch - 5'-Ende des Anker-Oligonukleotids durchgeführt werden, wobei durch das Anker-Oligonukleotid eine Tag-Sequenz am 3'-Ende der neu synthetisierten Nukleinsäure eingeführt wird. Bei einigen Ausführungsformen bedarf es einer Restriktionsschnittstelle in der Sequenz dieses ersten Primers. Das Anker-Oligonukleotid enthält bei dieser Ausführungsform zusätzlich in seiner Sequenz eine oder mehrere Restriktionsschnittstellen, wodurch eine intramolekulare Ligation des neu synthetisierten Nukleinsäurestrangs nach Restriktionsverdau ermöglicht werden kann.

Beispielsweise kann die Tag-Sequenz als Primer-Bindestelle für eine Rolling-Cirde-Amplification dienen. Somit kann das ganze Transkriptom oder auch das Genom amplifiziert werden (Fig. 11).

Es werden 0,1 µg total-RNA in einer RT-Reaktion mit Reverser Transkriptase, RT-Puffer, dATP, dCTP, dGTP, dTTP, 1 µM Oligo-dT-Primer (enthaltend die Sequenz einer NotI-Schnittstelle am 5'-Ende) und 10 µM eines Anker-Oligonukleotids nach dem Omniscript^{®} Standard-Protokoll (QIAGEN) In cDNA umgeschrieben. Das Anker-Oligonukleotid hat zum einen eine Zufallssequenz als hybridisierende Anker-Sequenz und zum anderen eine Tag-Template-Sequenz, die ebenfalls eine NotI-Schnittstelle enthält. Bei der cDNA-Synthese wird eine Tag-Sequenz mittels des Anker-Oligonukleotids am 3'-Ende der neu synthetisierten Nukleinsäure eingebaut, welche die NotI-Schnittstelle enthält. Nachfolgend wird der Zweitstrang der cDNA über eine Standard-Reaktion mit Klenow-Fragment, dNTPs und einem Primer, dessen Sequenz identisch zu einem Teil der Tag-Template-Sequenz des eingesetzten Anker-Oligonukleotids ist, erzeugt. Die cDNA wird über QIAquick^{®} (QIAGEN) nach Standard-Protokoll gereinigt und anschließend mit NotI geschnitten. Die DNA wird über QIAquick^{®} (QIAGEN) gereinigt und anschließend in einer 1000 µl Ligationsreaktion mit T4-Ligase ligiert, wobei überwiegend zirkuläre DNA-Moleküle entstehen. Die entstandenen zirkulären DNA-Moleküle werden mittels RCA vermehrt (eine schematische Darstellung findet sich in Fig. 11).

In Abwandlung zur beschriebenen Versuchsdurchführung kann z.B. ein Anker-Oligonukleotid mit spezifischer oder degenerierter Anker-Sequenz verwendet werden. In weiterer Abwandlung kann die cDNA-Synthese von Random-Primern oder spezifischen Primem gestartet werden, die an ihrem 5'-Ende eine Schnittstelle für ein Restriktionsenzym enthalten.

### Ausführungsform 9 (nicht zur Erfindung gehörend)

Auch diese Ausführungsform betrifft den Einsatz von Anker-Oligonukleotiden für die Einführung einer Schnittstelle für Restriktionsendonukleasen in eine Nukleinsäure zur Herstellung von Groß-Moleküle. Während Ausführungsform 8 jedoch zirkuläre DNA-Moleküle betrifft, ist Ausführungsform 9 mit der Herstellung linearer DNA -Moleküle befasst. Es kann DNA oder RNA als Ziel-Nukleinsäure verwendet werden. Hierzu kann ausgehend von einem ersten Primer die DNA-Synthese bis hin zum - nach Template Switch - 5'-Ende des Anker-Oligonukteotids durchgeführt werden, wobei durch das Anker-Oligonukleotid eine Tag-Sequenz am 3'-Ende der neu synthetisierten Nukleinsäure eingeführt wird. Bei einigen Ausführungsformen bedarf es einer Restriktionsschnittstelle in der Sequenz dieses ersten Primers. Das Anker-Oligonukleotid enthält bei dieser Ausführungsform zusätzlich in seiner Sequenz eine oder mehrere Restriktionsschnittstellen, wodurch eine intramolekulare Ligation des neu synthetisierten Nukleinsäurestrangs nach Restriktionsverdau ermöglicht werden kann (eine schematische Darstellung findet sich in Fig. 12).

Es wird 0,1 µg total-RNA in einer RT-Reaktion mit Reverser Transkriptase, RT-Puffer, dATP, dCTP, dGTP, dTTP, 1 µM Oligo-dT-Primer (enthaltend die Sequenz einer Noti-Schniftstelle am 5'-Ende) und 10 µM eines Anker-Oligonukleotids nach dem Omniscript^{®} Standard-Protokoll (QIAGEN) in cDNA umgeschrieben. Das Anker-Oligonukleotid hat zum einen eine Zufallssequenz als hybridisierende Anker-Sequenz und zum anderen eine Tag-Template-Sequenz, die ebenfalls eine NotI-Schnittstelle enthält. Bei der cDNA-Synthese wird eine Tag-Sequenz mittels des Anker-Oligonukleotids am 3'-Ende der neu synthetisierten Nukleinsäure eingeführt, welche die NotI-Schnittstelle enthält. Nachfolgend wird der Zweitstrang der cDNA über eine Standard-Reaktion mit Omniscript^{®}, dNTPs und einem Primer, dessen Sequenz identisch zu einem Teil der Tag-Template-Sequenz des eingesetzten Anker-Oligonukleotids ist, erzeugt. Die cDNA wird über QIAquick^{®} (QIAGEN) nach Standard-Protokoll gereinigt und anschließend mit NotI geschnitten. Das Reaktionsgemisch wird 5 min auf 65°C erhitzt, NotI somit inaktiviert. Ein Aliquot wird anschließend in einer 20 µl Ligationsreaktion mit T4-Ligase zu Konkatemeren ligiert. Die Ligationsprodukte werden in einer MDA nach dem Standard Repli-g^{®} Protokoll für gereinigte DNA (QIAGEN) amplifiziert.

In Abwandlung zur beschriebenen Versuchdurchführung kann z.B. ein Anker-Oligonucleotid mit spezifischer oder degenerierter Anker-Sequenz verwendet werden. In weiterer Abwandlung kann die cDNA-Synthese von Random-Primem oder spezifischen Primem gestartet werden.

### Ausführungsform 10 (nicht zur Erfindung gehörend)

Diese Ausführungsform betrifft den Einsatz von Anker-Oligonukleotiden für die Detektion von DNA in einer Real-Time PCR. Bei dieser Ausführungsform des erfindungsgemäßen Verfahrens wird mittels des Anker-Oligonukleotids eine Primer Bindestelle in die neu synthetisierte Nukleinsäure eingeführt. Die in die neu synthetisierte Nukleinsäure eingebaute Tag-Sequenz dient zusätzlich zur Primer-Bindestelle 3' von dieser als Sonden Bindestelle, sodass in einer nachfolgenden Real-Time PCR die synthetisierten Nukleinsäuremoleküle quantifiziert werden können. Es kann DNA oder RNA als Ziel-Nukleinsäure verwendet werden. Im Falle von RNA kann z.B. ausgehend von einem ersten Oligo-dT Primer, aufweisend ein Tag enthaltend eine Primer-Bindestelle, die DNA-Synthese bis hin zum - nach Template Switch - 5'-Ende des Anker-Oligonukleotids durchgeführt werden, wobei durch das Anker-Oligonukleotid eine Tag-Sequenz am 3'-Ende der neu synthetisierten Nukleinsäure eingeführt wird. Die Real-time PCR kann mittels eines SYBR Green RT-PCR Kits (QIAGEN) quantifiziert werden. In Abwandlung der Methode kann z.B. auch eine auf Sonden basierende Real-Time PCR durchgeführt werden, In Fig. 13 ist schematisch die vorbereitende Reaktion zur Real-time PCR-Analyse von cDNA dargestellt, bei der während der Synthese des neuen Nukleinsäurestrangs Sonden- und Primer-Bindestellen in den neu synthetisierten Strang eingeführt werden.

Es wird 0,01 µg total-RNA in einer RT-Reaktion mit Reverser Transkriptase, RT-Puffer, dNTP, 1 µM Oligo-dT-Primer (mit Primer-Bindestelle) und 1 µM eines Anker-Oligonukleotids nach einem Standard-RT Protokoll in cDNA umgeschrieben. Das Anker-Oligonukleotid enthält eine Zufallssequenz als hybridisierenden Anker und eine Tag-Template-Sequenz, wodurch eine Primer- und eine Sonden-Bindestelle am 3'-Ende der neu synthetisierten Nukleinsäure eingeführt wird. Anschließend wird in einer Real-time PCR mit QuantiTect^{®} Reagenzien (QIAGEN) und den Primern, die an den eingefügten Primer-Bindestellen hybridisieren, eine Real-Time PCR durchgeführt. Die Nukleinsäure kann durch Vergleich mit einem Nukleinsäurestandard während der Amplifikation quantifiziert werden.

In Abwandlung zur beschriebenen Versuchsdurchführung kann z.B. ein Anker-Oligonukleotid mit spezifischer oder degenerierter Sequenz verwendet werden. In weiterer Abwandlung kann die cDNA-Synthese von Random-Primern oder spezifischen Primem gestartet werden.

### Ausführungsform 11 (nicht zur Erfindung gehörend)

Diese Ausführungsform befasst sich mit Anker-Oligonukleotiden für die Fusion von Transkriptom- oder Genom-Fragmenten. Ziel der vorliegenden Verfahrensvariante ist, mittels der Tag-Template-Sequenzen der Anker-Oligonukleotide Fusionsstellen in die neu synthetisierte Nukleinsäure einzuführen, um zwei oder mehr neu synthetisierte Nukleinsäuren zu fusionieren. Dies kann z.B. bei evolutiven Verfahren oder auch SAGE zum Einsatz kommen. Es kann DNA oder RNA als Template-Nukleinsäure verwendet werden. In Fig. 14 wird schematisch gezeigt, wie die Tag-Template-Sequenz des Anker-Oligonukleotids komplementär in den neu synthetisierten Strang eingebaut wird. Ausgehend von einem ersten Oligo-dT Primer, der ein (AT)n-Tag enthält, und einem Anker-Oligonukleotid, das als Anker-Sequenz ein (N)n und als Tag-Template-Sequenz ebenfalls eine (AT)n-Sequenz enthält, werden (AT)n-Tags an den Enden der neu synthetisierten Nukleinsäuren, die miteinander hybridisieren können, hergestellt. Nach Hybridisierung der Enden zweier oder mehr neu synthetisierter Nukleinsäurestränge entstehen zirkuläre oder lineare Aggregate. In einer nachfolgenden Polymerase- und Ligase-Reaktion werden die einzelnen Moleküle linear kovalent miteinander verknüpft, so dass Fusionsmoleküle entstehen.

Es wird 0,01 µg total-RNA in einer Polymerase-Reaktion mit T4-Polymerase, Pol-Püffer" dNTP und 10 µM Anker-Oligonukleotid in Kontakt gebracht. Das Anker-Oligonukleotid dient in diesem speziellen Fall auch als Primer der Polymerase-Reaktion und hat die Sequenz (AT)₁₀N₈ (als, Tag-Template wird hier die (AT)₁₀-Sequenz und eine Random-Anker-Sequenz mit n=8 verwendet) eingesetzt. Die Reaktion findet für 60 min bei 37°C statt. Eine anschließende Erhitzung des Reaktionsgemisches auf 95°C für 5 min führt zur Denaturierung der DNA und zur Inaktivierung der Polymerase. Nach einer Re-Hybridisierung der DNA-Moleküle hybridisieren die AT-Enden miteinander. Durch die Zugabe einer DNA-Polymerase (T4 Polymerase) werden die Enden aufgefüllt. Es entstehen fusionierte DNA-Moleküle, die in einer anschließenden Ligationsreaktion mit T4-Ligase kovalent miteinander verbunden werden. Die entstehenden großen Moleküle werden über eine MDA (REPLI-g^{®}, QIAGEN) amplifiziert.

Die Erfindung wird nachfolgend anhand von Beispielen näher erläutert.

### Beispiel 1

Dieses Beispiel betrifft eine DNA-Amplifikation. Diese erfindungsgemäße Ausführungsform der Amplifikation beginnt mit denselben Schritten wie eine RNA-Amplifikation entsprechend Ausführungsform 2. Im vorliegenden Beispiel wurde hier neben der Tag-Sequenz am 3'-Ende der neu synthetisierten Nukleinsäure, die mittels Tag-Template-Sequenz des Anker-Oligonukleotids eingeführt wurde, weiterhin eine Tag-Sequenz am 5'-Ende der neu synthetisierten Nukleinsäure mittels eines Primers mit Tag-Sequenz an seinem 5'-Ende eingeführt. Die Tag-Sequenz des Primers sowie die Tag-Template-Sequenz des Anker-Oligonukleotids enthielten eine identische Sequenz. Dieses führte dazu, dass die Tag-Sequenzen am 3'-Ende und am 5'-Ende der neu synthetisierten Nukleinsäure komplementär zueinander sind. Nach Hybridisierung des 3'-Endes der neu synthetisierten Nukleinsäure mit dem komplementären 5'-Ende des neu synthetisierten Nukleinsäurestrangs bildete sich eine Haarnadelstruktur aus, die ein glattes doppelsträngiges. Ende enthielt. Zwei solcher Haamadelschlaufen wurden in einer "Blünt-end"-Ligation ligiert, so dass ein hantelförmiges, zirkulätes DNA-Molekül entstand. Dieses wurde mittels RCA amplifiziert (schematische Darstellung in Fig. 6).

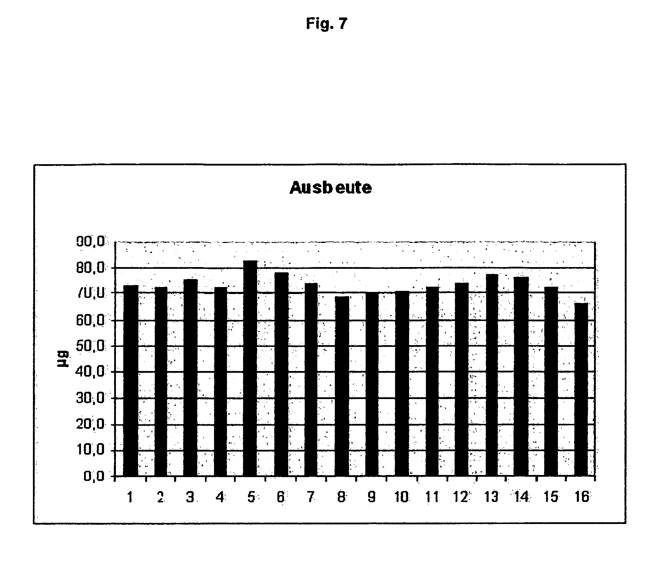

Je 100 ng einer RNA wurde in 16 unterschiedlichen Ansätzen in einer Polymerase-Reaktion mit der Reversen Transkriptase Sensiscripi^{®} (QIAGEN) umgesetzt. Die Polymerase-Reaktion wurde in Gegenwart eines geeigneten Puffers, Nukleotiden (0,5 mM) und Anker-Oligonukleotiden (10 µM) durchgeführt: Die Tag-Template-Sequenz der Anker-Oligonukleotide enthielt eine spezifische Sequenz (hier einen T7 RNA-Polymerase-Promotor) im 5'-Bereich und eine 8 Basen lange Zufallssequenz im 3'-Bereich. Nach einstündiger Reaktion bei 37°C wurde das Reaktionsgemisch für 5 min auf 95°C erhitzt. Anschließend wurde die DNA mittels einer Ligation (T4-Ligase) ligiert. Die entstandenen Ligationsprodukte wurden in einer RCA-Reaktion mit dem REPLI-g^{®} Kit (QIAGEN) amplifiziert. Hierbei wurde die Ausgangsmenge an cDNA stark amplifiziert, es wurden jeweils mehr als 60 µg DNA gebildet (siehe Fig. 7).

### Beispiel 2

Mit dem vorliegenden Beispiel wird gezeigt, dass die Tag-Template-Sequenz des Anker-Oligonukleotids als Template für die Synthese des 3'-Endes des neu synthetisierten Stranges dient. Die in diesem Versuch eingesetzten Anker-Oligonukleotide und Primer waren in ihrer Sequenz identisch. Die hybridisierenden Bereiche des Anker-Oligonukleotids und des Primers waren Random-Sequenzen von 8 Basen am 3'-Ende. Die Tag-Template-Sequenz der Anker-Oligonukleotids und die Tag-Sequenz des Primers wiesen beide die Täg7-Sequenz auf. Eine 20 µl Reverse Transkriptase-Reaktion wurde in einem RT-Puffer mit 100 ng total-RNA, 0,5 mM dNTPs, 10 U RNase Inhibitor (Promega), dem Sensiscript^{®} RT Kit (QIAGEN) und mit 10 µM Anker-Oligonukleotiden (Operon Biotechnologieä Inc.), die auch als Primer dienen, durchgeführt. Die Reaktion erfolgte für 60 min bei 37°C. Nach der Reverse Transkriptase Reaktion wurde der Ansatz geteilt. Eine Hälfte, der cDNA-Reaktionsansatzes wurde mit einem MinElitte Kit (QIAGEN) nach dem Cleanup Protokoll gereinigt, um die Anker-Oligonukleotide, die auch als Primer dienen, zu entfernen. Die gereinigte DNA wurde mit einem Volumen von 10 µl eluiert. In der Real-Tme PCR wurden die gereinigte und die ungereinigfe cDNA in gleicher Menge in verschiedenen Reaktionsgefäßen amplifiziert-und quantifiziert In diesem Versuch kamen Anker-Oligonukleotide mit der Sequenz
aat tct aat acg act cac tat agg gag aag gnn nnn nnn
zum Einsatz. Die Real-Time PCR wurde mit dem Primer
aat tct aat acg act cac tat agg
durchgeführt, Die Real-Time PCR wurde in einem ABI Prism 7700 nach dem QuantiTect SYBR Green Protokoll (QIAGEN) durchgeführt. In Fig. 15 ist die Real-Time PCR Analyse der PCR Fragmente zu erkennen. Mit beiden neu synthetisierten Nukleinsäuresträngen (gereinigt oder ungereinigt) konnte der gleiche CT-Wert erreicht werden.

Dies bedeutet, dass die Tag-Template-Sequenz des Anker-Oligonukleotids nicht erst während der Zweitstrang-Synthese (hier durchgeführt während der PCR) eingebaut wurde, da das Anker-Oligonukleotid bei der gereinigten Probe während der PCR aufgrund der Reinigung nicht mehr im Reaktionsgemisch vorhanden war. Somit wurde bereits während der Synthese des neu synthetisieren Stranges die Tag7-Sequenz durch die Reverse Transkriptase mittels Template-Switch eingebaut.

### Beispiel 3

Dieser Versuch zeigte, dass die Tag-Sequenz am 3'-Ende des neu synthetisierten Nukleinsäurestrangs mittels der Tag-Template-Sequenz des Anker-Oligonukleotids auch mit blockiertem 3'-Ende, d.h. nicht durch eine Polymerase verlängerbares 3'-Ende, eingebaut wird. Das heißt, dass die Tag-Template-Sequenz durch Template-Switch als Template bei der Synthese des neuen Nukleinsäurestranges diente und nicht das Anker-Oligonukleotid als Primer für die Zweitstrang-Synthese Verwendung fand.

Eine 20 µl Reverse Transkriptase-Reaktion wurde mit 100 ng total-RNA, 0,5 mM dNTPs, 10 U RNase Inhibitor (Promega), Sensiscript (QIAGEN), 1 µM β-act-Primer (Operon) und 10 µM Anker-Oligonukleotid (Operon) nach Sensiscript Standärdprotokoll durchgeführt: Die Reaktion erfolgte für 60 min bei 37°C. Das Anker-Oligonukleotid trug ein blockiertes 3'-Ende (inverse Base G = dG-ref-Q), das nicht verlängerbar ist, und enthielt weiterhin eine Zufallssequenz von 8 Basen am 3'-Ende (Anker-Sequenz) und eine Tag-Template-Sequenz (hier. die T7P-Sequenz) am 5'-Ende.
T7P-N8-block: AATTCTAATACGACTCACTTATAGGGAGAAGGNNNNNNNN-dG-ref-Q
T7P-N8: CAATTCTAATACGACTCACTATAGGGAGAAGGNNNNNNNNG
T7P: AATTCTAATACGACTCACTATAGGGAGAAGG
ß-act: GTCTCAAGTCAGTGTACAGG

Im Vergleich wurde der gleiche Versuch mit einem nicht-blockierten Anker-Oligonukleotid mit ansonsten identischer Sequenz durchgeführt.

Die in der RT-Reaktion generierte cDNA wurde in unterschiedlichen PCRs amplifiziert, wobei (a) β-act- und T7P-Primer oder (b) nur β-act-Primer zugeführt wurde. Die PCR folgte dem Standard-Protokoll für eine Endpunkt-PCR nach dem Taq PCR Handbuch von QIAGEN. In Fig. 16 ist die Gelanalyse der PCR-Fragmente zu erkennen. Als Marker wurde eine Kb-Leiter (1 kb Leiter, Invitrogen GmbH) verwendet. cDNA-Amplifikate in einer Größe von bis zu 350 bp waren nur zu erkennen, wenn beide Primer (β-act und T7P-Primer) in der PCR eingesetzt wurden. Dabei spielte es keine Rolle, ob die cDNA-Synthese mit blockierten oder nicht-blockierten Anker-Oligonukleotiden durchgeführt wurde: Mit beiden Anker-Oligonukleotiden (blockiertes 3'-Ende und nicht-blockiertes 3'-Ende) wurden cDNA-Amplifikate erhalten. Es wurden hingegen keine Amplifikate erzeugt, wenn die PCR nur mit β-act-Primern durchgeführt wurde.

Dies bedeutet, dass die Tag-Sequenz nicht während der Zweitstrang-Synthese durch Verlängerung des Anker-Oligonukleotids, sondern bereits während der Erststtang-cDNA-Synthese mittels Template-Switch durch die Reverse Transkriptase in die neu synthetisierte Nukleinsäure eingebaut wurde, da ein freies 3'-OH-Ende am Anker-Oligonukleotid, das für eine entsprechende Zweitstrang-Synthese gebraucht würde, nicht notwendig war.

### Beispiel 4

Mit diesem Beispiel wurd geklärt, dass unterschiedlich lange Anker-Sequenzen am 3'-Ende des Anker-Oligonukleotids mit unterschiedlicher Effizienz an Template-Nukleinsäure hybridisieren können.

Eine Reverse Transkriptase-Reaktion wurde mit 100 ng total-RNA oder 0 ng total-RNA (Negativkontrolle), 0,5 mM dNTPs; 10 U RNase Inhibitor (Promega), 4 U Omniscript RT (QIAGEN) und 10 µM Anker-Oligonukleotide (Operon) durchgeführt. Die Anker-Oligonukleotide dienten im vorliegenden Versuch auch gleichzeitig als Primer. Die Reaktion erfolgte für 60 min bei 37°C. Die Anker-Oligonukleotide trugen ein freies 3'-OH Ende und enthielten weiterhin eine Zufallssequenz von 8 oder mehr Basen am 3'-Ende (Anker-Sequenz) und eine Tag-Template-Sequenz (T7).

Es wurden verschiedene Anker-Oligonukleotide mit unterschiedlich langen Ankersequenzen verwendet (N₈, N₁₀, N₁₂, wobei N= A,C,G, oder T ist). Die neu synthetisierte Nukleinsäure wurde in einer PCR mit Primem amplifiziert, die nur die T7-Sequenz enthielten. Die PCR folgte einem Standard-Protokoll für eine Endpunkt-PCR nach dem Taq PCR Handbuch von QIAGEN. In einer weiteren PCR wurde dieselbe neu synthetisierte Nukleinsäure mit Primern amplifiziert, die spezifisch das β-Aktin-Transkript erkennen. Folgende Oligonukleotide fanden Verwendung:

| | |
|---|---|
| T7: | gga tga cga cgc agt att g |
| ß-act-Primer: | gtctcaagtcagtgtacagg |
| | gtgatagcattgctttcgtg |
| T7N8: | gga tga cga cgc agt att g nnn nnn nn |
| T7N10: | gga tga cga cgc agt att g nnn nnn nnn n |
| T7N12: | gga tga cga cgc agt att g nnn nnn nnn nnn |

In Fig. 17 ist die Gelanalyse der PCR Fragmente zu erkennen. Als Größenmarker wurde die 1 Kb-Leiter (siehe Beispiel 3) verwendet. In der Negativkontrolle waren keine Amplifikate zu erkennen, da während der Reverse Transkriptase Reaktion keine RNA, vorhanden war. Die Amptifikation mit T7-Primem führte zu verschieden großen Fragmenten, die eine Größe von 200 bp bis 1500 bp aufwiesen, wobei sowohl die Größe als auch die Menge der Schar größer wurde; wenn die Länge der Anker-Sequenz-zunahm. Mit den ß-act Primem wurde ein Nukleinsäurefragment der erwarteten Größe amplifiziert.

Daraus lässt sich schließen, dass Anker-Oligonukleotide sowohl als solche als auch gleichzeitig als Primer eingesetzte werden können, um die Tag-Sequenz am 3'-Ende wie auch am 5'-Ende der neu synthetisierten Nukleinsäure einzuführen. Weiterhin ergibt sich, dass je länger die Random Anker-Sequenz ist, desto weniger häufig hybridisieren diese spezifisch mit der Template-Nukleinsäure, da die statistische Häufigkeit der jeweiligen Sequenz in der Schar der Random Anker-Sequenzen der Anker-Oligonukleotide abnimmt. Darüber hinaus hybridisiert eine längere Random Anker-Sequenz jedoch auch effizienter mit der Template-Nukleinsäure. Dies wird sichtbar an der Menge und der Länge der Amplifikate, die durch eine PCR mit Primem der T7-Sequenz erhalten wurden (Fig. 17).

### Beispiel 5

Mit diesem Beispiel wird geklärt, dass unterschiedliche Reverse Transkriptasen den Einbau von Tag-Sequenzen am 3'-Ende der neu synthetisierten Nukleinsäure mittels der Tag-Template-Sequenzen von Anker-Oligonukleotiden über Template-Switch vermitteln.

Reverse Transkriptase-Reaktionen wurden mit 100 ng total-RNA oder 1 ng total-RNA, 0,5 mM dNTPs, 10 U RNase Inhibitor (Promega), verschiedenen Reversen Transkriptasen und 10 µM Anker-Oligonukleotide (Operon) durchgeführt. Die Anker-Oligonukleotide (T7N6T4) dienten im vorliegenden Versuch auch gleichzeitig, als Primer. Die Reaktion erfolgte für 60 min bei 37°C. Die Anker-Oligonukleotide trugen ein freies 3'-OH Ende und enthielten weiterhin eine Zufallssequenz von 8 Basen gefolgt von 4 T am 3'-Ende (Anker-Sequenz) und eine Tag-Template-Sequenz (T7).

Als Reverse Transkriptasen wurden Omniscript (QIAGEN), Sensiscript (QIAGEN), MMLV RT (Invitrogen) bzw. MMLV RT ohne RNase H-Aktivität (Süperscript II, in Fig. 18 als SSII abgekürzt; Invitrogen) eingesetzt. Pro durchgeführte Reaktion wurde jeweils nur eine Reverse Transkriptase verwendet.

Die neu synthetisierte Nukleinsäure wurde in einer PCR mit Primern amplifiziert, die nur die T7-Sequenz enthielten. Die PCR folgte einem Standard-Protokoll für eine Endpunkt-PCR nach dem Taq PCR Handbuch von QIAGEN. In einer weiteren PCR wurde dieselbe neu synthetisierte Nukleinsäure mit Primern amplifiziert, die spezifisch das ß-Aktin-Transkript erkennen. Folgende Oligonukleotide fanden Verwendung:

| | |
|---|---|
| T7: | gga tta cga ctc agt att g |
| T7N6T4: | gga tta cga ctc agt att g nnnnnn tttt |
| ß-act-Primer: | gtctcaagtcagtgtacagg |
| | gtgatagcattgctttcgtg |

In Abb. 18 ist die Gelanalyse der PCR Fragmente zu erkennen. Als Größenmarker wurde die 1 Kb-Leiter (siehe Beispiel 3) verwendet. Die Amplifikation mit T7-Primern führte zu verschieden großen Fragmenten, die eine Größe von ungefähr 300 bis 2500 bp aufwiese. Mit den ß-act-Primern wurde ein Fragment der erwarteten Größe amplifiziert. Mit allen Reversen Transkriptasen konnte die Tag-Sequenz erfolgreich in die Erststrang-cDNA eingebaut werden.

Dies lässt den Schluss zu, dass verschiedene Reverse Transkriptasen den Einbau von Tag-Sequenzen in neu synthetisierte Nukleinsäuren mittels der Tag-Template-Sequenz der Anker-Oligonukleotide vermitteln können.

### Beispiel 6

Anhand dieses Beispiels wurde gezeigt, dass der Abstand zwischen den Anker-Oligonukleotid-Hybridisierungsstellen moduliert werden kann. Dabei wurden Tag-Template-Sequenzen von Anker-Oligonukleotiden für die Einführung der Tag-Sequenz in den neu synthetisierten Nukleinsäurestrang verwendet.

Eine Reverse Transkriptase-Reaktion wurde mit 0 ng (Negativkontrolle), 1 ng bzw. 100 ng total-RNA, 0,5 mM dNTPs, 10 U RNase Inhibitor, 4 U Omniscript^{®} (QIAGEN) und verschiedenen Anker-Oligonukleotiden (jeweils 10 µM) durchgeführt. Die Anker-Oligonukleotide dienten in diesem speziellen Falle auch als Primer. Die Reaktion erfolgte für 30 min bei 37°C. Die Anker-Oligonukleotide trugen jeweils ein freies 3'-OH Ende und wiesen eine Random Anker-Sequenz von 6 Basen Länge auf, die zwischen der am 3'-Ende liegenden Tₙ-Sequenz (mit n = 0, 1, 2 oder 3) und einer Tag-Template-Sequenz (hier genannt T7) am 5'-Ende liegt. Die Primer unterschieden sich in der Länge der Oligo-T-Region am 3'-Ende, die zwischen 0 und 3 liegt (T0, T1, T2, T3).

Der neu synthetisierte Nukleinsäurestrang wurde in einer PCR mit Primern amplifiziert, die mit der T7-Sequenz hybridisierten. Die PCR folgte einem Standard-Protokoll für eine Endpunkt-PCR nach dem Taq PCR Handbuch von QIAGEN. In einer weiteren PCR wurde der gleiche neu synthetisierte Nukleinsäurestrang mit Primem amplifiziert, die spezifisch das ß-Aktin-Transkript erkennen. Folgende Oligonukleotide fanden Verwendung:

| | |
|---|---|
| T7: | gga tga cga cgc agt att g |
| T3: | gga tga cga cgc agt att g nnnnnn ttt |
| T2: | gga tga cga cgc agt att g nnnnnn tt |
| T1: | gga tga cga cgc agt att g nnnnnn t |
| T0: | gga tga cga cgc agt att g nnnnnn |
| ß-act-Primer: | gtctcaagtcagtgtacagg |
| | gtgatagcattgctttcgtg |

In Fig. 19 ist die Gelanalyse der PCR-Fragmente zu erkennen. Als Marker wurde die 1 Kb-Leiter verwendet (siehe Beispiel 3). Die Amplifikation mit T7-Primem führte zu einer Schar verschieden großer Fragmente, die eine Größe von ca. 300 bis 2500 bp aufwiesen. Mit den ß-act-Primem wurde ein Fragment erwarteter definierter Größe amplifiziert. Mit allen in diesem Beispiel verwendeten Anker Oligonukleotiden konnte mittels der Tag-Template-Sequenzen die Tag-Sequenz erfolgreich in den neu synthetisierten Nukleinsäurestrang eingebaut werden. Dies zeigt sich durch die erfolgreiche Amplifikation der neu synthetisierten Nükleinsäurestränge mittels Primern, die an der Tag-Sequenz hybridisieren (siehe Fig. 19). Mit Zunahme der Spezifität der Anker-Sequenz mit Erhöhung der Anzahl an Thymin-Basen am 3'-Ende der Anker-Oligonukleotide wurden diese Amplifikate im Schnitt größer. Zudem nahm mit zunehmender Spezifität die Menge an Primer-Dimer-Produkten ab (siehe in Fig. 19 die Reaktionsprodukte in den Ansätzen '0 ng', die keine RNA in der RT-Reaktion enthalten).

Daraus lässt sich schließen, dass die Erhöhung der Spezifität der Anker-Sequenz den Abstand der Hybridisierung der Anker-Oligonukleotide vergrößert. Auch wird durch die Erhöhung der Spezifität der Anker-Sequenz die Menge an unspezifischem Primer-Dimer-Produkt reduziert.

### Beispiel 7

Anhand dieses Beispiels wurde die Effizienz des Einbaus der Tag-Sequenz mittels der Tag-Template-Sequenz von Anker-Oligonukleotiden in einer Real-Time PCR untersucht. Dabei wurden Tag-Template-Sequenzen von Anker-Oligonukleotiden für die Einführung der Tag-Sequenz in den neu synthetisierten Nukleinsäurestrang verwendet.

Eine Reverse Transkriptase-Reaktion wurde mit 100 ng total-RNA, 0,5 mM dNTPs, 10 U RNase Inhibitor (Promega), Sensiscript^{®} (QIAGEN) und verschiedenen Anker-Oligonukleotiden (jeweils 10 µM) Primern (Operon) nach Sensiscript Kit Handbuch (QIAGEN) durchgeführt. Die Reaktion erfolgte für 30 min. bei 37°C.

Alle Anker-Oligonukleotide trugen jeweils ein freies 3'-OH Ende und enthielten weiterhin eine Zufallssequenz als Anker-Sequenz im 3'-Bereich und eine Tag-Template-Sequenz (gga tga cga cgc agt att g) im 5'-Bereich. Die neu synthetisierte Nukleinsäure wurde in einer Real-Time PCR amplifiziert und quantifiziert. Als Primer wurden hier Oligonukleotide eingesetzt, die an der Tag-Sequenz hybridisieren. Folgende Oligonukleotide kamen zum Einsatz:

| | |
|---|---|
| T7: | gga tga cga cgc agt att g |
| N8 | gga tga cga cgc agt att g nnn nnn nn |
| N10: | gga tga cga cgc agt att g nnn nnn nnn n |
| N12: | gga tga cga cgc agt att g nnn nnn nnn nnn |
| G2N6: | gga tga cga cgc agt att ggg nnn nnn |
| G4N6: | gga tga cga cgc agt att ggg gg nnn nnn |

In Fig. 20 ist die Real-Time PCR-Analyse der PCR-Fragmente zu erkennen. Die Real-Time PCR folgte einem Protokoll des QuantiTect SYBR Green Kits von QIAGEN. Mit allen Anker-Oligonukleotiden konnte die Tag-Sequenz erfolgreich in den neu synthetisierten Nukleinsäurestrang eingebaut werden. Die entstandenen neu synthetisierten Nukleinsäurestränge konnten erfolgreich mittels Primern, die an der Tag-Sequenz der neu synthetisierten Nukleinsäure hybridisierten, in der Real-Time PCR Analyse analysiert werden.

Daraus lässt sich erkennen, dass die Effizienz des Einbaus der Tag-Sequenz in den neu synthetisierten Nukleinsäurestrang sich durch Real-Time PCR quantifizieren lässt. Eine Verlängerung der Random Anker-Sequenz auf 12 Basen führte in diesem speziellen Versuch zu einer verbesserten Effizienz des Einbaus (siehe Fig. 20, Primer N12).

### Beispiel 8

Durch dieses Beispiel konnte gezeigt werden, dass eine Amplifikation des Gesamtgenoms (Whole Genome Amplification) mittels des erfindungsgemäßen Verfahrens durchgeführt werden kann. Hierbei dient gDNA als Template-Nukleinsäure. Auch in diesem Beispiel wurden Tag-Sequenzen in einer Polymerase-Reaktion mittels der Tag-Template-Sequenzen der Anker-Oligonukleotide in den neu synthetisierten Nukleinsäurestrang eingebaut.

Als Anker-Oligonukleotid wurde das Anker-Oligonukleotid T7P-N8 verwendet. Als Tag-Template-Sequenz enthält das Anker-Oligonukleotid am 5'-Ende eine T7-Promotor-Sequenz. Der 3'-Bereich enthielt eine 8 Basen lange Zufallssequenz.

Zunächst wurde 120 ng gDNA aus HeLa-Zellen in Gegenwart von T7P-N8 Oligonukleotid (10 µM), Reaktionspuffer und dNTPs (0,5 mM) bei 95°C für 5 min denaturiert. Alternativ wurde anstelle des T7P-N8 Oligonukleotids ein blockiertes, (d.h. mit durch eine Polymerase nicht verlängerbarem 3'-Ende) Anker-Oligonukleotid mit derselben Sequenz (T7P-N8-block) bzw. in einer weiteren Alternative ein 8 Basen langer Random Primer eingesetzt.
T7P-N8:
CAA TTC TAA TAC GAC TCA CTA TAG GGA GAA GGN NNN NNN N
T7P-N8-Block:
CAA TTC TAA TAC GAC TCA CTA TAG GGA GAA GGN NNN NNN N -dG-ref-Q.
N8:
NNN NNN NN
dG-ref-Q ist eine invers orientierte Guanin-Base, die durch eine Polymerase nicht verlängert werden kann.

Nach der Denaturierung der DNA wurde das Reagenziengemisch auf Raumtemperatur abgekühlt. Anschließend wurden 4 U MMLV (RNase H minus) (SuperscriptII, Invitrogen) hinzugegeben und die Ansätze für 1 h bei 37°C inkubiert. Anschließend wurde 1 µl des Reaktionsgemischs in eine PCR überführt. Die PCR wurde für 40 Zyklen in einem 20 µl Ansatz in Gegenwart eines Primer durchgeführt, der mit der Tag-Sequenz des neu synthetisierten Nukleinsäurestrangs hybridisieren kann (im vorliegenden Falle Primer T7P), dNTPs, PCR-Puffer und Taq-Polymerase durchgeführt.
T7P:
CAA TTC TAA TAC GAC TCA CTA TAG GGA GAA GG

Die Ergebnisse dieses Beispiels zeigen, dass ein Einbau der Tag-Sequenz durch ein Anker-Oligonukleotid möglich war, und zwar auch dann, wenn DNA als Matrize (Template) verwendet wird. Eine Whole Genome Amplification ist mit dem erfindungsgemäßen Verfahren also möglich. Ein Einbau der Tag-Sequenz in die neu synthetisierte Nukleinsäure war nicht möglich, wenn ein Anker-Oligonukleotid mit blockiertem 3'-Ende verwendet wird, da das Anker-Oligonukleotid in diesem Fall bei der Polymerase-Reaktion ebenfalls als Primer fungierte (siehe Fig. 21).

### Beispiel 9

Mit Hilfe dieses Beispiels wurde gezeigt, dass neben Reverse Transkriptasen auch andere Polymerasen den Einbau der Tag-Sequenz in den neu synthetisierten Nukleinsäurestrang durch die Tag-Template-Sequenz des Anker-Oligonukleotids vermitteln können.

Hierzu wurde das Anker-Oligonukleotid T7P-N8 verwendet (Sequenz: CAA TTC TAA TAC GAC TCA CTA TAG GGA GAA GGN NNN NNN N)). Zunächst wurden 10 ng gDNA aus HeLa-Zellen in Gegenwart von T7P-N8 Oligonukleotid (10 µM), 1x-Klenow-Puffer und dNTPs (0,5 mM) bei 95°C für 5 min denaturiert. Alternativ wurde ein N8 Primer (Sequenz: NNN NNN NN) verwendet. Das denaturierte Reagenziengemisch wurde auf Raumtemperatur abgekühlt. Anschließend wurde 1 µl Sensiscript (QIAGEN), 12 U AMV Reverse Transkriptase, oder 10 U Klenow-Fragment von DNA Polymerase I aus *E.coli* zu den einzelnen Ansätzen hinzugegeben und für 1 h bei 37°C inkubiert. Anschließend wurde 1 µl des Reaktionsgemischs in eine PCR überführt. Es wurden 40 PCR-Zyklen in einem 20 µl Ansatz in Gegenwart von T7P-Primer (Sequenz: CAA TTC TAA TAC GAC TCA CTA TAG GGA GAA GG), dNTPs, Primer, PCR-Puffer und Taq-Polymerase durchgeführt.

Die Versuchsergebnisse (siehe Fig. 22) dieses Beispiels zeigen, dass auch andere Polymerasen, in diesem Falle das Klenow-Fragment der DNA-Polymerase 1, den Einbau einer Tag-Sequenz in den neu synthetisierten Nukleinsäurestrang durch die Tag-Template-Sequenz eines Anker-Oligonukleotids vermitteln können.

## Patentansprüche

1. Verfahren zur Einführung mehrerer Tag-Sequenzen in eine Nukleinsäure, umfassend die folgenden Schritte:
(a) Bereitstellen von Template-Nukleinsäure;
(b) Hybridisieren der Anker-Sequenz von Anker-Oligonukleotiden, wobei die Anker-Oligonukleotide im 3'-Bereich eine zur Template-Nukleinsäure komplementäre Anker-Sequenz und im 5'-Bereich mindestens eine spezifische Sequenz (Tag-Template-Sequenz) aufweisen, mit einem Sequenzabschnitt der Template-Nukleinsäure;
(c) Synthetisieren neuer Nukleinsäurestränge, welche partiell komplementär zur Template-Nukleinsäure sind und welche an ihrem 3'-Ende eine zu dem nicht-hybridisierenden Teil des Anker-Oligonukleotids, d.h. der Tag-Template-Sequenz, komplementäre Sequenzen enthalten, wodurch am 3'-Ende der neu synhetisierten Nukleinsäurestränge doppelsträngige Nukleinsäurebereiche entstehen; und
(d) weitere Prozessierung der in Schritt (c) erzeugten doppelsträngigen Nukleinsäurestränge,
**dadurch gekennzeichnet, dass**
die Synthese der neuen Nukleinsäurestränge aus Schritt (c) von Anker-Oligonukleotiden ausgeht, wobei in Schritt (c) eine Polymerase mit geringer oder keiner Strand-Displacement-Aktivität eingesetzt wird, so dass, wenn die Polymerase auf ein Anker-Oligonukleotid stößt, die weitere Synthese entlang des nicht-hybridisierenden Bereichs des Anker-Oligonukleotids erfolgt und die Polymerase von der Template-Nukleinsäure auf das Anker-Oligonukleotid überspringt, und dass weiterhin die Prozessierung aus Schritt (d) die folgenden Schritte umfasst:
(i) die Ligation der in Schritt (c) entstehenden doppelsträngigen Enden der neu synthetisierten Nukleinsäurestränge, ohne dass eine vorherige Strangtrennung erfolgt, und nachfolgend
(ii) die Trennung des Doppelstranges, wobei sich zirkuläre einzelsträngige Nukleinsäuren bilden.

2. Verfahren nach Anspruch 1, **dadurch gekennzeichnet, dass** die Tag-Template-Sequenz des Anker-Oligonukleotids aus Schritt (b) eine funktionelle Sequenz ausgewählt aus Hybridisierungsstellen, Primer-Bindestellen, Sonden-Bindestellen, Promotoren, Signalsequenzen zur Initiation der Transkription und/oder Translation, Restriktionsendonuklease-Erkennungs- und -Schnitt-Stellen, Ribosomen-Bindestellen, Protein-Bindestellen, Antikörper-Erkennungsstellen, oder die zu diesen komplementären Sequenzen aufweist.

3. Verfahren gemäß Anspruch 1 oder 2, **dadurch gekennzeichnet, dass** der in Schritt (c) neu synthetisierte Nukleinsäurestrang vor Schritt (d) zusätzlich mittels einer sequenzspezifischen Endonuklease prozessiert wird.

4. Verfahren gemäß Anspruch 3, **dadurch gekennzeichnet, dass** die sequenzspezifische Endonuklease eine methylierungssensitive Endonuklease ist.

5. Verfahren nach einem der Ansprüche 1 bis 4, **dadurch gekennzeichnet, dass** die entstandenen zirkulären einzelsträngigen Nukleinsäuren mittels Rolling Circle Amplifikation amplifiziert werden.

6. Verfahren nach Anspruch 2, **dadurch gekennzeichnet, dass** die funktionelle Sequenz ein RNA-Polymerase Promotor ist.

7. Verfahren nach Anspruch 6, **dadurch gekennzeichnet, dass** nachfolgend eine *in vitro* Transkription durchgeführt wird.

## Claims

1. Method of introducing several tag sequences into a nucleic acid, comprising the following steps:
(a) providing a template nucleic acid;
(b) hybridizing the anchor sequence of anchor oligonucleotides, the anchor oligonucleotide having in the 3' region an anchor sequence complementary to the template nucleic acid and in the 5' region at least one specific sequence (tag-template sequence), with one sequence section of the template nucleic acid;
(c) synthesizing new nucleic acid strands which are partially complementary to the template nucleic acid and which contain at their 3' end sequences complementary to the non-hybridizing part of the anchor oligo-nucleotide, i.e. the tag-template sequence, thereby producing double-stranded nucleic acid regions on the 3' end of the newly synthesized nucleic acid strands; and
(d) further processing of the double-stranded nucleic acid strands generated in step (c),
**characterized in that**
synthesis of the new nucleic acid strands in step (c) starts from anchor oligonucleotides, with a polymerase with low or no strand displacement activity being employed in step (c), such that upon said polymerase encountering an anchor oligonucleotide further synthesis is performed along the non-hybridizing region of the anchor oligo-nucleotide, the polymerase switching from the template nucleic acid to the anchor oligo-nucleotide, and that furthermore processing in step (d) comprises the following steps:
(i) ligation of the double-stranded ends forming in step (c) of the newly synthesized nucleic acid strands, without any previous strand separation, and subsequently
(ii)separation of the double strand, with circular single-stranded nucleic acids being produced.

2. Method according to Claim 1, **characterized in that** the tag-template sequence of the anchor oligonucleotide in step (b) has a functional sequence selected from hybridization sites, primer binding sites, probe binding sites, promoters, signal sequences for initiating transcription and/or translation, restriction endonuclease recognition and cleavage sites, ribosome binding sites, protein binding sites, antibody recognition sites, or the sequences complementary thereto.

3. Method according to Claim 1 or 2, **characterized in that** the nucleic acid strand newly synthesized in step (c) is additionally processed by means of a sequence-specific endonuclease prior to step (d).

4. Method according to Claim 3, **characterized in that** the sequence-specific endonuclease is a methylation-sensitive endonuclease.

5. Method according to any of Claims 1 to 4, **characterized in that** the resulting circular single-stranded nucleic acids are amplified by means of rolling circle amplification.

6. Method according to Claim 2, **characterized in that** the functional sequence is an RNA polymerase promoter.

7. Method according to Claim 6, **characterized in that** an *in vitro* transcription is carried out subsequently.

## Revendications

1. Procédé pour introduire plusieurs séquences étiquettes dans un acide nucléique, comprenant les étapes suivantes :
(a) mise à disposition d'une matrice d'acide nucléique,
(b) hybridation de la séquence d'ancrage des oligonucléotides d'ancrage, les oligonucléotides d'ancrage présentant dans le domaine 3' une séquence d'ancrage complémentaire de la matrice d'acide nucléique et dans le domaine 5' au moins une séquence spécifique (séquence étiquette-matrice), ayant un segment de séquence de la matrice d'acide nucléique ;
(c) synthèse de nouveaux brins d'acides nucléiques, qui sont partiellement complémentaires de la matrice d'acide nucléique, et qui en leurs extrémités 3' contiennent une séquence complémentaire de la partie non hybridable de l'oligonucléotide d'ancrage, c'est-à-dire la séquence étiquette-matrice, ce qui crée des domaines d'acides nucléiques double brin à l'extrémité 3' des brins d'acides nucléiques néosynthétisés, et
(d) maturation plus poussée des brins d'acides nucléiques double brin produits dans l'étape (c),
**caractérisé en ce que** la synthèse des nouveaux brins d'acides nucléiques de l'étape (c) part des oligonucléotides d'ancrage, une polymérase ayant une activité faible ou nulle de déplacement de brin étant utilisée dans l'étape (c), de telle sorte que, quand la polymérase heurte un oligonucléotide d'ancrage, la suite de la synthèse ait lieu le long du domaine non hybridant de l'oligonucléotide d'ancrage et la polymérase saute de la matrice d'acide nucléique à l'oligonucléotide d'ancrage, et en outre que la maturation de l'étape (d) comprend les étapes suivantes :
(i) la ligature des extrémités double brin générées dans l'étape (c) des brins d'acides nucléiques néosynthétisés, sans qu'il se produise un séparation préalable des brins, et ensuite
(ii) la séparation du double brin, à l'occasion de laquelle des acides nucléiques simple brin circulaires se forment.

2. Procédé selon la revendication 1, **caractérisé en ce que** la séquence étiquette-matrice de l'oligonucléotide d'ancrage de l'étape (b) présente une séquence fonctionnelle choisie parmi les sites d'hybridation, les sites de fixation de l'amorce, les sites de liaison à des sondes, les promoteurs, les séquences signal pour l'initiation de la transcription et/ou de la traduction, les sites de reconnaissance et de clivage par des endonucléases de restriction, les sites de liaison du ribosome, les sites de liaison des protéines, les sites de reconnaissance d'anticorps, ou les séquences qui leur sont complémentaires.

3. Procédé selon la revendication 1 ou 2, **caractérisé en ce que** le brin d'acide nucléique néosynthétisé dans l'étape (c) subit en outre, avant l'étape (d), une maturation à l'aide d'une endonucléase spécifique de séquence.

4. Procédé selon la revendication 3, **caractérisé en ce que** l'endonucléase spécifique de séquence est une endonucléase sensible à la méthylation.

5. Procédé selon l'une des revendications 1 à 4, **caractérisé en ce que** les acides nucléiques circulaires simple brin créés sont amplifiés par amplification par cercle roulant.

6. Procédé selon la revendication 2, **caractérisé en ce que** la séquence fonctionnelle est un promoteur de l'ARN-polymérase.

7. Procédé selon la revendication 6, **caractérisé en ce qu'**on procède à une transcription in vitro.


### SEQUENCE LISTING

<110> QIAGEN GmbH
<120> Insertion of Sequence Elements into Nucleic acids
<130> PA140-EP-DIV
<150> DE 10 2006 020 885.4
   <151> 2006-05-05
<160> 23
<170> PatentIn version 3.3
<210> 1
   <211> 39
   <212> DNA
   <213> artificial sequence
<220>
   <223> oligonucleotide with an 8 base random sequence at the 3' end
<220>
   <221> misc_feature
   <222> (32)..(39)
   <223> n is a, c, g, or t
<400> 1
   aattctaata cgactcacta tagggagaag gnnnnnnnn 39
<210> 2
   <211> 27
   <212> DNA
   <213> artificial sequence
<220>
   <223> oligonucleotide with an 8 base random sequence at the 3' end
<220>
   <221> misc_feature
   <222> (20)..(27)
   <223> n is a, c, g, or t
<400> 2
   ggatgacgac gcagtattgn nnnnnnn 27
<210> 3
   <211> 29
   <212> DNA
   <213> artificial sequence
<220>
   <223> oligonucleotide with an 10 base random sequence at the 3' end
<220>
   <221> misc_feature
   <222> (20)..(29)
   <223> n is a, c, g, or t
<400> 3
   ggatgacgac gcagtattgn nnnnnnnnn 29
<210> 4
   <211> 31
   <212> DNA
   <213> artificial sequence
<220>
   <223> oligonucleotide with an 12 base random sequence at the 3' end
<220>
   <221> misc_feature
   <222> (20)..(31)
   <223> n is a, c, g, or t
<400> 4
   ggatgacgac gcagtattgn nnnnnnnnnn n 31
<210> 5
   <211> 24
   <212> DNA
   <213> artificial sequence
<220>
   <223> primer
<400> 5
   aattctaata cgactcacta tagg 24
<210> 6
   <211> 25
   <212> DNA
   <213> artificial sequence
<220>
   <223> oligonucleotide with a 6 base random sequence at the 3' end
<220>
   <221> misc_feature
   <222> (20)..(25)
   <223> n is a, c, g, or t
<400> 6
   ggatgacgac gcagtattgn nnnnn 25
<210> 7
   <211> 26
   <212> DNA
   <213> artificial sequence
<220>
   <223> oligonucleotide with a 6 base random sequence and a 1 t sequence at the 3' end
<220>
   <221> misc_feature
   <222> (20)..(25)
   <223> n is a, c, g, or t
<400> 7
   ggatgacgac gcagtattgn nnnnnt 26
<210> 8
   <211> 27
   <212> DNA
   <213> artificial sequence
<220>
   <223> oligonucleotide with a 6 base random sequence and a 2 t base sequence at the 3' end
<220>
   <221> misc_feature
   <222> (20)..(25)
   <223> n is a, c, g, or t
<400> 8
   ggatgacgac gcagtattgn nnnnntt 27
<210> 9
   <211> 28
   <212> DNA
   <213> artificial sequence
<220>
   <223> oligonucleotide with a 6 base random sequence and a 3 t base sequence at the 3' end
<220>
   <221> misafeature
   <222> (20)..(25)
   <223> n is a, c, g, or t
<400> 9
   ggatgacgac gcagtattgn nnnnnttt 28
<210> 10
   <211> 31
   <212> DNA
   <213> artificial sequence
<220>
   <223> T7 promoter
<400> 10
   aattctaata cgactcacta tagggagaag g 31
<210> 11
   <211> 20
   <212> DNA
   <213> artificial sequence
<220>
   <223> ß-actin primer
<400> 11
   gtctcaagtc agtgtacagg 20
<210> 12
   <211> 19
   <212> DNA
   <213> artificial sequence
<220>
   <223> primer
<400> 12
   ggatgacgac gcagtattg 19
<210> 13
   <211> 27
   <212> DNA
   <213> artificial sequence
<220>
   <223> oligonucleotide with a 6 base random sequence at the 3' end
<220>
   <221> misc_feature
   <222> (22)..(27)
   <223> n is a, c, g, or t
<400> 13
   ggatgacgac gcagtattgg gnnnnnn 27
<210> 14
   <211> 29
   <212> DNA
   <213> artificial sequence
<220>
   <223> oligonucleotide with a 6 base random sequence at the 3' end
<220>
   <221> misc_feature
   <222> (24)..(29)
   <223> n is a, c, g, or t
<400> 14
   ggatgacgac gcagtattgg gggnnnnnn 29
<210> 15
   <211> 19
   <212> DNA
   <213> artificial sequence
<220>
   <223> T7 sequence
<400> 15
   ggattacgac tcagtattg 19
<210> 16
   <211> 29
   <212> DNA
   <213> artificial sequence
<220>
   <223> oligonucleotide with a 6 base random sequence and a 4 t base sequence at the 3' end
<220>
   <221> misc_feature
   <222> (20)..(25)
   <223> n is a, c, g, or t
<400> 16
   ggattacgac tcagtattgn nnnnntttt 29
<210> 17
   <211> 8
   <212> DNA
   <213> artificial sequence
<220>
   <223> random primer
<220>
   <221> misc_feature
   <222> (1)..(8)
   <223> n is a, c, g, or t
<400> 17
   nnnnnnnn 8
<210> 18
   <211> 20
   <212> DNA
   <213> artificial sequence
<220>
   <223> ß-actin primer
<400> 18
   gtgatagcat tgctttcgtg 20
<210> 19
   <211> 40
   <212> DNA
   <213> artifitial sequence
<220>
   <223> oligonucleotide with an 8 base random sequence at the 3' end
<220>
   <221> misc_feature
   <222> (33)..(40)
   <223> n is a, c, g, or t
<400> 19
   caattctaat acgactcact atagggagaa ggnnnnnnnn 40
<210> 20
   <211> 32
   <212> DNA
   <213> artificial sequence
<220>
   <223> primer
<400> 20
   caattctaat acgactcact atagggagaa gg 32
<210> 21
   <211> 40
   <212> DNA
   <213> Artificial Sequence
<220>
   <223> oligonucleotide with an 8 base Random sequence and an inverse base G at the 3' end
<220>
   <221> misc_feature
   <222> (32)..(39)
   <223> n is a, c, g, or t
<220>
   <221> modified_base
   <222> (40)..(4C)
   <223> nucleotide that inhibits primer elongation
<400> 21
   aattctaata cgactcacta tagggagaag gnnnnnnnng 40
<210> 22
   <211> 41
   <212> DNA
   <213> artificial sequence
<220>
   <223> oligonucleotide with an 8 base random sequence and a g base at the 3' end
<220>
   <221> misc_feature
   <222> (33)..(40)
   <223> n is a, c, g, or t
<400> 22
   caattctaat acgactcact atagggagaa ggnnnnnnnn g 41
<210> 23
   <211> 41
   <212> DNA
   <213> artificial sequence
<220>
   <223> oligonucleotide with an 8 base random sequence and an invers g base at the 3' end
<220>
   <221> misc_feature
   <222> (33)..(40)
   <223> n is a, c, g, or t
<220>
   <221> modified_base
   <222> (41)..(41)
   <223> nucleotide that inhibits primer elongation
<400> 23
   caattctaat acgactcact atagggagaa ggnnnnnnnn g 41